# EUROPEAN PATENT APPLICATION

(11) **EP 4 623 809 A2**
(43) Date of publication of application: **01.10.2025**
(21) Application number: 25190726.7
(22) Date of filing: 30.03.2023
(51) Int. Cl.: A61B 5/00

(54) **SYSTEMS AND METHODS FOR VASCULAR IMAGE CO-REGISTRATION**

(30) Priority: 30.03.2022 US 202263325349 P
(62) Divisional of application: 23719184.6
(71) Applicant: Boston Scientific Scimed, Inc., Maple Grove, MN 55311 (US)
(72) Inventor: LI, Wenguang, Los Gatos, California 95032 (US); BLOMS, Kevin, Minneapolis, Minnesota 55419 (US); HONG, Zhichao, Maple Grove, Minnesota 55311 (US); TORBORG, Alan, Minneapolis, Minnesota 55416 (US); ZINGSHEIM, Brandon, Circle Pines, Minnesota 55014 (US)
(74) Representative: Vossius & Partner Patentanwälte Rechtsanwälte mbB

(57) **Abstract**

The present application relates to a method for simultaneously displaying co-registered extravascular and intravascular images along-side one another on a display (150) for an intravascular imaging system (102), the method comprising obtaining intravascular image data from an intravascular imaging system/sub-system (106) and obtaining extravascular image data from an extravascular imaging system/sub-system (104), wherein the extravascular imaging data and the intravascular imaging data are obtained during a translation procedure, co-registering the intravascular imaging data with the extravascular imaging data to create co-registered extravascular imaging data and co-registered intravascular imaging data, displaying a first image output (153) showing the co-registered extravascular imaging data upon a display (150). displaying a second image output (154) showing the co-registered intravascular imaging data upon the display (150), and displaying a second slider bar or cursor (600) on the second image output (154) and a corresponding first slider bar or cursor (602) on the first image output, (153).

## Description

### Cross-Reference To Related Applications

This application claims the benefit of priority of U.S. Provisional Application No. 63/325,349, filed March 30, 2022, the entire disclosure of which is hereby incorporated by reference.

### Technical Field

The present disclosure pertains to medical imaging, and systems and methods for medical imaging. More particularly, the present disclosure pertains to systems and methods for vascular imaging including intravascular imaging and extravascular imaging and co-registration.

### Background

A wide variety of medical imaging systems and methods have been developed for medical use, for example, use in imaging vascular anatomy. Some of these systems and methods include intravascular imaging modalities and extravascular imaging modalities for imaging vasculature. These systems and methods include various configurations and may operate or be used according to any one of a variety of methods. Of the known vascular imaging systems and methods, each has certain advantages and disadvantages. Accordingly, there is an ongoing need to provide alternative systems and methods for vascular imaging and assessment, and co-registration of imaging.

### Brief Summary

This disclosure provides alternative medical imaging systems and methods. An example includes a method for determining a position of interest for performing a medical procedure. The method includes obtaining extravascular imaging data of a portion of a blood vessel, the extravascular imaging data including an extravascular image showing an intravascular imaging device disposed within the vessel, with an imaging element of the intravascular imaging device disposed at a starting location for a translation procedure during which the imaging element is translated within the blood vessel from the starting location to an ending location. An extravascular contrast image showing the portion of the blood vessel with contrast and showing a visualized anatomical landmark is obtained. Intravascular imaging data is obtained from the intravascular imaging device during the translation procedure, the intravascular imaging data including one or more intravascular images showing a detected anatomical landmark. The detected anatomical landmark and the visualized anatomical landmark are used to co-register the intravascular imaging data with the extravascular imaging data. A position of interest within the vessel between the starting location and the ending location is determined. The position of interest is displayed on a live fluoroscopy session display.

Another example includes a method for determining a position for performing a medical procedure. The method includes obtaining extravascular imaging data of a portion of a blood vessel, the extravascular imaging data including an extravascular image showing a starting location and an ending location for a translation procedure during which an intravascular imaging element is translated within the blood vessel from the starting location to the ending location. Intravascular imaging data is obtained from the intravascular imaging device during the translation procedure. The intravascular imaging data is co-registered with the extravascular imaging data to create co-registered imaging data. The co-registered imaging data is used to determine a position of interest within the portion of the blood vessel between the starting location and the ending location. The position of interest is displayed on a live fluoroscopy session display.

Alternatively or additionally, determining a position of interest within the vessel between the starting location and the ending location may include automatically determining a position of interest based on the intravascular imaging data.

Alternatively or additionally, the method may further include displaying the co-registered intravascular imaging data and extravascular imaging data.

Alternatively or additionally, the position of interest on live fluoroscopy may be displayed in an intravascular imaging console via a real-time data connection from the fluoroscopy machine.

Alternatively or additionally, the position of interest displayed on live fluoroscopy may be automatically synchronized with cardiac motion via ECC gating.

Alternatively or additionally, the position of interest displayed on live fluoroscopy may be automatically synchronized with motion analysis of a radiopaque object such as a guide catheter or a guide wire.

Alternatively or additionally, determining a position of interest within the vessel between the starting location and the ending location may include studying the displayed co-registered intravascular imaging data and extravascular imaging data.

Alternatively or additionally, the medical procedure may include implanting a stent.

Alternatively or additionally, displaying the position of interest on a live fluoroscopy display may include displaying a distal marker indicating a target location for a distal end of the stent.

Alternatively or additionally, displaying the position of interest on a live fluoroscopy display may further include displaying a proximal marker indicating a target location for a proximal end of the stent.

Alternatively or additionally, displaying the position of interest on a live fluoroscopy display may further include providing the position of interest to the live fluoroscopy machine via a data link with the live fluoroscopy display.

Alternatively or additionally, the data link may include a bi-directional data link.

Alternatively or additionally, the method may further include communicating data from the live fluoroscopy display to a display that is displaying the co-registered intravascular imaging data and extravascular imaging data.

Alternatively or additionally, the data link may include the Internet.

Alternatively or additionally, the extravascular imaging data may include one or both of angiographic image data and fluoroscopic image data.

Alternatively or additionally, the intravascular imaging data may include one or more of intravascular ultrasound data and optical coherence tomography data.

Alternatively or additionally, the translation procedure may be a pullback.

Another example includes a method for determining a position of interest for performing a medical procedure. The method includes obtaining extravascular imaging data for a portion of a blood vessel and obtaining intravascular imaging data for the same portion of the blood vessel. The intravascular imaging data is co-registered with the extravascular imaging data. A position of interest for performing a medical procedure is determined and the position of interest is displayed on a live fluoroscopy session display.

Alternatively or additionally, the medical procedure may include implanting a stent.

Alternatively or additionally, displaying the position of interest on a live fluoroscopy display may include displaying a distal marker indicating a target location for a distal end of the stent.

Alternatively or additionally, displaying the position of interest on a live fluoroscopy display may further include displaying a proximal marker indicating a target location for a proximal end of the stent.

Another example includes a method for determining a position of interest for implanting a stent. The method includes obtaining extravascular imaging data for a portion of a blood vessel and obtaining intravascular imaging data for the same portion of the blood vessel. The intravascular imaging data is co-registered with the extravascular imaging data. A desired implantation location for implanting a stent is determined, and is displayed on a live fluoroscopy session display.

Alternatively or additionally, displaying the desired stent implantation location may include displaying one or more of a distal marker indicating a target location for a distal end of the stent and a proximal marker indicating a target location for a proximal end of the stent.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a schematic illustration of an exemplary system for use in vascular imaging co-registration;
FIG. 2 is a schematic illustration of an exemplary intravascular imaging catheter, shown in partial cross-sectional view;
FIG. 3 is a schematic illustration of the distal portion of the exemplary intravascular imaging catheter of FIG. 2, shown in cross-section;
FIG. 4 is a schematic illustration of a display showing an example extravascular image;
FIG. 5 is a schematic illustration of the display showing an example extravascular contrast image;
FIG. 6. is a schematic illustration of the display showing an example extravascular image, and showing corresponding intravascular imaging including transverse cross-sectional and longitudinal cross-sectional images, obtained during a beginning portion of a translation procedure;
FIG. 7 is a schematic illustration of the display showing an example extravascular image, and showing corresponding intravascular imaging including transverse cross-sectional and longitudinal cross-sectional images, obtained during an intermediate portion of a translation procedure;
FIG. 8 is a schematic illustration of the display showing an example extravascular image, and showing corresponding intravascular imaging including transverse cross-sectional and longitudinal cross-sectional images, obtained during an ending portion of a translation procedure;
FIG. 9 is a schematic illustration of the display showing an example combined extravascular image marked with beginning and ending locations of a translation procedure, and showing corresponding intravascular imaging including transverse cross-sectional and longitudinal cross-sectional images;
FIG. 10 is a schematic illustration of a display showing an example extravascular image including a marked predicted location of a detected anatomical landmark, and showing corresponding intravascular imaging including transverse cross-sectional and longitudinal cross-sectional images;
FIG. 11 is a schematic illustration of a display as in FIG. 10, showing the marked predicted location of a detected anatomical landmark aligned with a visualized anatomical landmark, and showing corresponding intravascular imaging including transverse cross-sectional and longitudinal cross-sectional images;
FIG. 12 is a schematic illustration of a display showing an example extravascular image including a marked predicted location of second detected anatomical landmark, and showing corresponding intravascular imaging including transverse cross-sectional and longitudinal cross-sectional images;
FIG. 13 is a schematic illustration of a display as in FIG. 12, showing the marked predicted location of the second detected anatomical landmark aligned with a second visualized anatomical landmark, and showing corresponding intravascular imaging including transverse cross-sectional and longitudinal cross-sectional images;
FIG. 14 is a schematic illustration of a display showing an example extravascular image including a marked predicted location of third detected anatomical landmark, and showing corresponding intravascular imaging including transverse cross-sectional and longitudinal cross-sectional images;
FIG. 15 is a schematic illustration of a display as in FIG. 14, showing the marked predicted location of the third detected anatomical landmark aligned with a third visualized anatomical landmark, and showing corresponding intravascular imaging including transverse cross-sectional and longitudinal cross-sectional images;
FIG. 16 is a schematic illustration of a display showing an example extravascular image including a marked predicted location of fourth detected anatomical landmark, and showing corresponding intravascular imaging including transverse cross-sectional and longitudinal cross-sectional images;
FIG. 17 is a schematic illustration of a display as in FIG. 16, showing the marked predicted location of the fourth detected anatomical landmark aligned with a fourth visualized anatomical landmark, and showing corresponding intravascular imaging including transverse cross-sectional and longitudinal cross-sectional images;
FIG. 18 is a schematic illustration of a display showing co-registered images including an example extravascular image, and showing corresponding intravascular imaging including transverse cross-sectional and longitudinal cross-sectional images;
FIG. 19 is a schematic illustration of an exemplary catheter lab;
FIG. 20 is an exemplary live fluoroscopy image;
FIG. 21 is an exemplary live fluoroscopy image;
FIG. 22 is a schematic illustration of a display showing an exemplary live fluoroscopy image, and showing corresponding intravascular imaging including transverse cross-sectional and longitudinal cross-sectional images; and
FIG. 23 is a schematic illustration of a display showing co-registered images including an example extravascular image, and showing corresponding intravascular imaging including schematically shown transverse cross-sectional and longitudinal cross-sectional images in combination with an interactive slider mechanism.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the disclosure.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "or" unless the content clearly dictates otherwise.

It is noted that references in the specification to "an embodiment", "some embodiments", "other embodiments", etc., indicate that the embodiment described may include one or more particular features, structures, or characteristics. However, such recitations do not necessarily mean that all embodiments include the particular features, structures, or characteristics. Additionally, when particular features, structures, or characteristics are described in connection with one embodiment, it should be understood that such features, structures, or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

A number of different medical imaging modalities may be used to evaluate or treat blood vessels. Two general types of imaging modalities include extravascular imaging modalities and intravascular imaging modalities. This disclosure relates to the use and co-registration of these modalities.

Extravascular imaging modalities, such as various forms of radiological imaging, provide extravascular imaging data of a portion of a blood vessel. Some examples include angiography or fluoroscopy imaging modalities, such as two-dimensional angiography/fluoroscopy; three-dimensional angiography/fluoroscopy; or computer tomography angiography/fluoroscopy. Angiography typically involves rendering a radiological view of one or more blood vessels, often with the use of radiopaque contrast media. An angiographic image can also be viewed real time by fluoroscopy. In general, fluoroscopy uses less radiation than angiography, and is often used to guide medical devices including radiopaque markers within or through vessels. Extravascular imaging data of blood vessels may provide useful information about the blood vessel, the anatomy or the location or positioning of devices within the blood vessel or anatomy. For example, extravascular imaging data (e.g. angiograms) may provide a comprehensive overall image or series of images or a video of the blood vessel(s) of interest, and may provide a "roadmap" with a good temporal resolution for the general assessment of the blood vessel(s) or navigation of devices within blood vessels.

Intravascular imaging modalities provide intravascular imaging data of a portion of a blood vessel. Some examples of intravascular imaging modalities include intravascular ultrasound (IVUS) and optical coherence tomography (OCT). These modalities typically include imaging the vessel itself using a device-mounted intravascular probe including an imaging element disposed within the vessel. Several types of device systems have been designed to track through a vasculature to provide intravascular image data. These can include, but are not limited to, intravascular ultrasound (IVUS) devices and optical coherence tomography (OCT) devices (e.g. catheters, guidewires, etc.) In operation, intravascular device-mounted probes including an imaging element are moved along a blood vessel in the region where imaging is desired. As the probe passes through an area of interest, sets of intravascular image data are obtained that correspond to a series of "slices" or cross-sections of the vessel, the lumen, and surrounding tissue. These devices may include radiopaque material or markers. Such markers are generally positioned near a distal tip or near or on the probe. Therefore, the approximate location of the imaging probe or imaging element can be discerned by observing the procedure on either a fluoroscope or an angiographic image or images. Typically, such imaging devices are connected to a dedicated processing unit or control module, including processing hardware and software, and a display. The raw image data is received by the console, processed to render an image including features of concern, and rendered on the display device. Intravascular imaging data of blood vessels may provide useful information about the blood vessel that is different from or in addition to the information provided by the extravascular imaging data. For example, intravascular imaging data may provide data regarding the cross-section of the lumen, the thickness of deposits on a vessel wall, the diameter of the non-diseased portion of a vessel, the length of diseased sections, the makeup of deposits or plaque on the wall of the vessel, assessment of plaque burden or assessment of stent deployment.

These two general types of imaging modalities provide different imaging data, and therefore may be complimentary to each other. As such, in certain circumstances, it may be desirable to provide or use both general types of medical imaging modalities to evaluate or treat blood vessels. Additionally, it may be useful for the locations of the acquired intravascular imaging data/images to be correlated with their locations on the vessel roadmap obtained by the extravascular imaging data/images. It may be useful to coordinate or "register" (e.g. co-register) the imaging data rendered by the two different modalities. It may also be useful to display the co-registered extravascular imaging data and intravascular imaging data together, for example, on a common display monitor. Some example embodiments disclosed herein may include or relate to some or all of these aspects.

In accordance with some embodiments of the present disclosure, example method(s), system(s), device(s), or software are described herein. These examples include image data acquisition equipment and data/image processors, and associated software, for obtaining and registering (e.g. co-registering) imaging data rendered by the two distinct imaging modalities (e.g. extravascular imaging data and intravascular imaging data). Additionally, or alternatively, example method(s), system(s) or software may generate views on a single display that simultaneously provides extravascular images with positional information and intravascular images associated with an imaging probe (e.g., an IVUS or OCT probe) mounted upon an intravascular device.

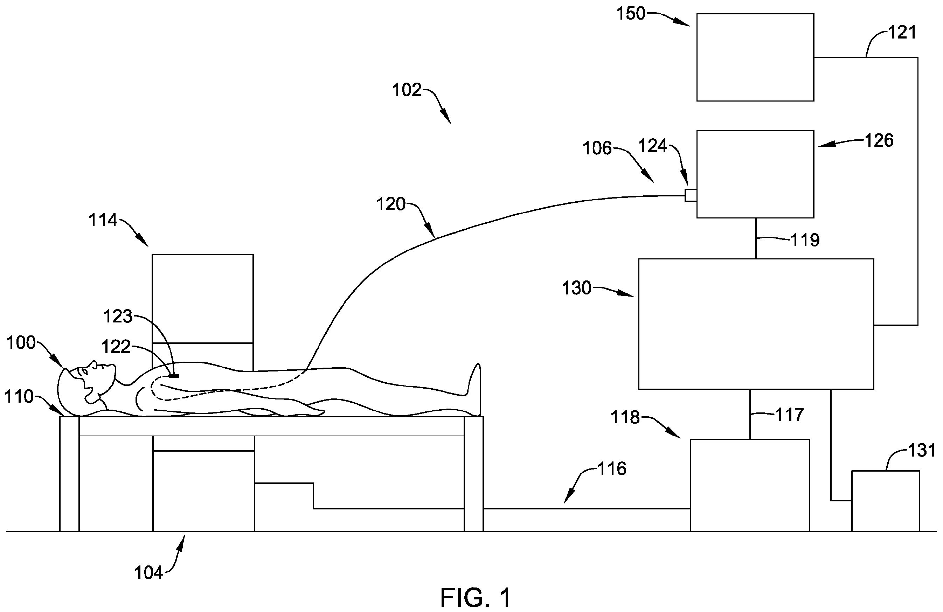

FIG. 1 is schematic depiction of an exemplary system 102 that may be used in conjunction with carrying out an embodiment of the present disclosure through obtaining and co-registering extravascular image data (e.g. angiogram/fluoroscopy) and intravascular image data (e.g. IVUS or OCT images). The system 102 may include an extravascular imaging system/sub-system 104 (e.g. angiography/fluoroscopy system) for obtaining/generating extravascular imaging data. The system 102 may also include an intravascular imaging system/sub-system 106 (e.g. IVUS or OCT) for obtaining/generating intravascular imaging data. The system 102 may include a computer system/sub-system 130 including one or more controller or processor, memory and/or software configured to execute a method for vascular imaging registration of the obtained extravascular imaging data and the obtained intravascular imaging data.

The extravascular imaging data may be radiological image data obtained by the angiography/fluoroscopy system 104. Such angiography/fluoroscopy systems are generally well known in the art. The angiography/fluoroscopy system 104 may include an angiographic table 110 that may be arranged to provide sufficient space for the positioning of an angiography/fluoroscopy unit c-arm 114 in an operative position in relation to a patient 100 on the table 110. Raw radiological image data acquired by the angiography/fluoroscopy c-arm 114 may be passed to an extravascular data input port 118 via a transmission cable 116. The input port 118 may be a separate component or may be integrated into or be part of the computer system/sub-system 130. The angiography/fluoroscopy input port 118 may include a processor that converts the raw radiological image data received thereby into extravascular image data (e.g. angiographic/fluoroscopic image data), for example, in the form of live video, DICOM, or a series of individual images. The extravascular image data may be initially stored in memory within the input port 118, or may be stored within the computer 130. If the input port 118 is a separate component from the computer 130, the extravascular image data may be transferred to the computer 130 through the cable 117 and into an input port in the computer 130. In some alternatives, the communications between the devices or processors may be carried out via wireless communication, rather than by cables.

The intravascular imaging data may be, for example, IVUS data or OCT data obtained by the intravascular imaging system/sub-system 106 (e.g. an IVUS or OCT system). Such IVUS and OCT systems are generally well known in the art. The intravascular sub-system 106 may include an intravascular imaging device such as an imaging catheter 120, for example an IVUS or OCT catheter. The imaging device 120 is configured to be inserted within the patient 100 so that its distal end, including a diagnostic assembly or probe 122 (e.g. an IVUS or OCT probe), is in the vicinity of a desired imaging location of a blood vessel. A radiopaque material or marker 123 located on or near the probe 122 may provide indicia of a current location of the probe 122 in a radiological image.

By way of example, in the case of IVUS intravascular imaging data, the diagnostic probe 122 generates ultrasound waves, and receives ultrasound echoes representative of a region proximate the diagnostic probe 122. The probe 122 or catheter 120 may convert the ultrasound echoes into corresponding signals, such as electrical or optical signals. The corresponding signals are transmitted along the length of the imaging catheter 120 to a proximal connector 124. The proximal connector 124 of the catheter 120 is communicatively coupled to processing unit or control module 126. IVUS versions of the probe 122 come in a variety of configurations including single and multiple transducer element arrangements. It should be understood that in the context of IVUS, a transducer may be considered an imaging element. In the case of multiple transducer element arrangements, an array of transducers is potentially arranged: linearly along a lengthwise axis of the imaging catheter 120, curvilinearly about the lengthwise axis of the catheter 120, circumferentially around the lengthwise axis, etc.

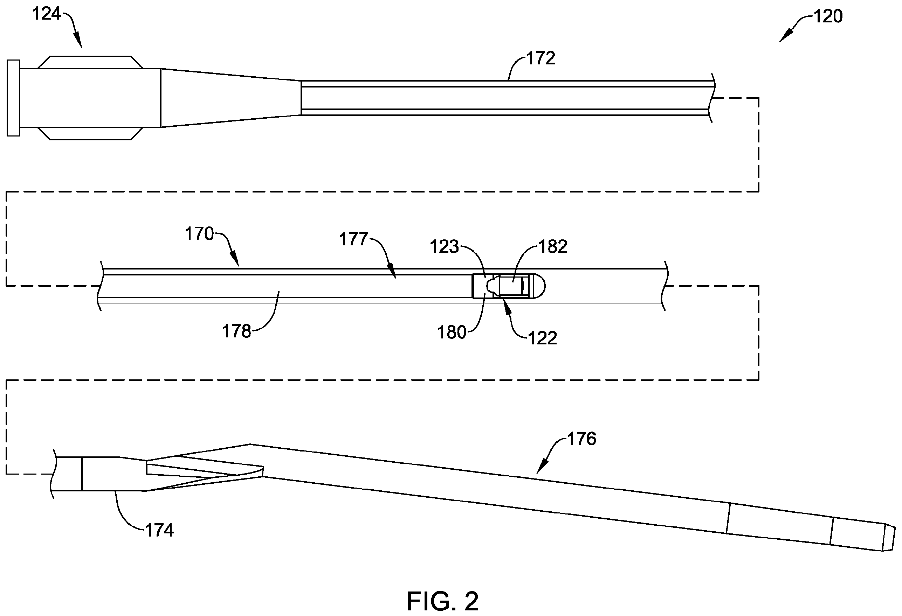

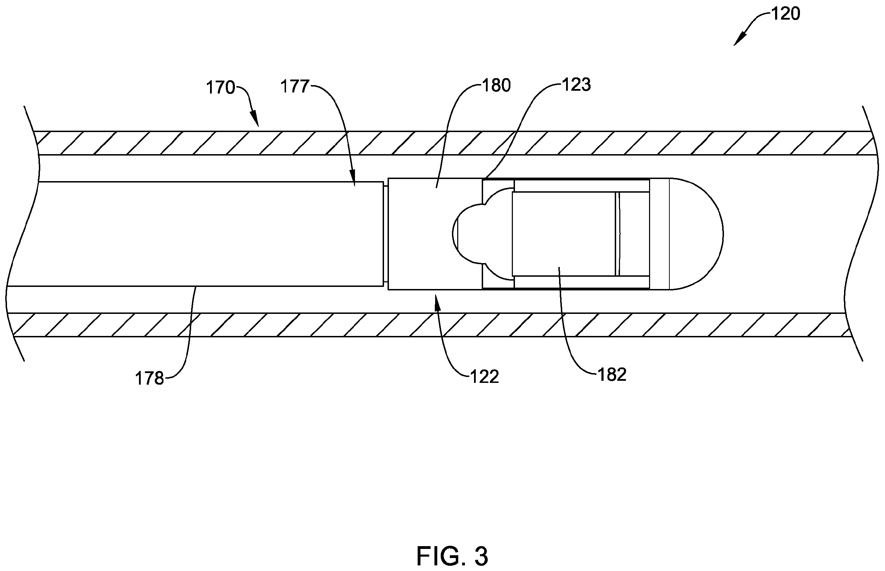

One example of an IVUS intravascular imaging catheter 120 is shown in FIGS. 2 and 3. The imaging catheter 120 may include an elongate shaft 170 having a proximal end region 172 and a distal end region 174. The proximal hub or connector 124 may be coupled to or otherwise disposed adjacent to the proximal end region 172. A tip member 176 may be coupled to or otherwise disposed adjacent to the distal end region 174. The tip member 176 may include a guidewire lumen, an atraumatic distal end, one or more radiopaque markers, or other features. An imaging assembly 177 may be disposed within the shaft 170. In general, the imaging assembly 177 (which may include an imaging probe 122 including an imaging element182) may be used to capture/generate images of a blood vessel. In some instances, the medical device may include devices or features similar to those disclosed in U.S. Patent Application Pub. No. US 2012/0059241 and U.S. Patent Application Pub. No. US 2017/0164925, the entire disclosures of which are herein incorporated by reference. In at least some instances, the medical device 120 may resemble or include features that resemble the OPTICROSS^{™} Imaging Catheter, commercially available from BOSTON SCIENTIFIC, Marlborough, MA.

As shown in FIG. 3, the imaging assembly 177 may include a drive cable or shaft 178, an imaging probe 122 including a housing 180 and an imaging element or transducer 182. The imaging probe 122 or housing 180 may be coupled to the drive cable 178. The transducer 182 may be rotatable or axially translatable relative to the shaft 170. For example, the drive cable 178 may be rotated or translated in order to rotate or translate the transducer 182. The probe 122 or housing 180, for example, may include or be made of a radiopaque material or marker 123, which may provide indicia of a current location of the probe 122 in a radiological image.

Referring back to FIG. 1, by way of another example, the device 120 may be an OCT catheter used to collect OCT intravascular data. The OTC catheter 120 may include a diagnostic probe 122 that generates or propagates a light beam that is directed at tissue, and a portion of this light that reflects from sub-surface features is collected and is representative of a region proximate the diagnostic probe 122. In OCT, the diagnostic probe 122 will include an optical imager for delivery and collection of the light. It should be understood that in the context of OCT, the optical imager in the probe 122 may be considered an imaging element. A technique called interferometry may be used to record the optical path length of received photons allowing rejection of most photons that scatter multiple times before detection. Thus, OCT can build up images of thick samples by rejecting background signal while collecting light directly reflected from surfaces of interest. The probe 122 or catheter 120 may transmit the optical or light signals along the shaft, or may convert light signals into corresponding signals, such as electrical or optical signals, that may be transmitted along the length of the imaging catheter 120 to a proximal connector 124. The proximal connector 124 of the catheter 120 is communicatively coupled to a processing unit or control module 126. The probe 122 or housing 180, may include or be made of a radiopaque material or marker 123, which may provide indicia of a current location of the probe 122 in a radiological image

Raw intravascular image data (e.g. raw IVUS or OCT data) may be acquired by the imaging catheter 120 and may be passed to the control module 126, for example via connector 124. The control module 126 may be a separate component or may be integrated into or be part of the computer system/sub-system 130. The control module 126 may include a processor that converts or is configured to convert the raw intravascular image data received via the catheter 120 into intravascular image data (e.g. IVUS or OCT image data), for example, in the form of live video, DICOM, or a series of individual images. The intravascular imaging data may include transverse cross-sectional images of vessel segments. Additionally, the intravascular imaging data may include longitudinal cross-sectional images corresponding to slices of a blood vessel taken along the blood vessel's length. The control module 126 may be considered an input port for the computer system/subsystem 130, or may be considered to be connected to an input port of the computer 130, for example, via cable 119 or a wireless connection. The intravascular image data may be initially stored in memory within the control module 126, or may be stored within memory in the computer system/subsystem 130. If the control module 126 is a separate component from the computer system/sub-system 130, the intravascular image data may be transferred to the computer 130, for example through the cable 119, and into an input port in the computer 130. Alternatively, the communications between the devices or processors may be carried out via wireless communication, rather than by cable 119.

The control module 126 may also include one or more components that may be configured to operate the imaging device 120 or control the collection of intravascular imaging data. For example, in the case of an IVUS system, the control module 126 may include one or more of a processor, a memory, a pulse generator, a motor drive unit, or a display. As another example, in the case of an OCT system, the control module 126 may include one or more of a processor, a memory, a light source, an interferometer, optics, a motor drive unit, or a display. In some cases, the control module 126 may be or include a motor drive unit that is configured to control movement of the imaging catheter 120. Such a motor drive unit may control rotation or translation of the imaging catheter 120 or components thereof. In some instances, the control module 126 or motor drive unit may include an automatic translation system that may be configured to translate the imaging catheter 120 in a controlled/measured matter within the patient 100. Such an automatic translation system may be used such that during a translation procedure, the imaging catheter 120 (including an imaging element) is translated within the blood vessel from a starting location to an ending location at a constant or known speed. (e.g. the imaging catheter 120 is translated at a specific rate for a known amount of time). In other embodiments, the translation may be done manually. Translation procedures may be, for example, a "pullback" procedure (where the catheter 120 is pulled through the vessel) or a "push-through" procedure (where the catheter 120 is pushed through the vessel). The control module 126 may also be configured from or include hardware and software configured to control intravascular imaging and data collection. For example, the control module 126 may include control features to turn on/off imaging or data collection from/to the catheter 120.

The computer system/sub-system 130 can include one or more controller or processor, one or more memory, one or more input port, one or more output port and/or one or more user interface. The computer 130 obtains or is configured to obtain intravascular image data from or through the intravascular imaging system/sub-system 106 (e.g. IVUS or OCT) and extravascular image data from or through the extravascular imaging system/sub-system 104 (e.g. angiography/fluoroscopy system). The computer 130, or the components thereof, can include software and hardware designed to be integrated into standard catheterization procedures and automatically acquire both extravascular imaging data (e.g. angiography/fluoroscopy) and intravascular imaging data (e.g. IVUS or OCT) through image or video acquisition.

The computer system/sub-system 130, or the components thereof, can include software or hardware that is configured to execute a method for vascular imaging co-registration of the obtained extravascular imaging data and the obtained intravascular imaging data. In that context, the computer 130 may include computer readable instructions or software to execute the method for vascular imaging co-registration as disclosed herein. For example, in some respects the computer may include a processor or a memory which includes software including program code causing the computer to execute the method for vascular imaging co-registration as disclosed herein. For example, the computer/computing device can include a processor or memory including instructions executable by the processor to perform the method for vascular imaging co-registration as disclosed herein. In that context, it can also be appreciated that also disclosed herein is a computer readable medium having stored thereon in a non-transitory state a program code for use by the computer/computing device 130, the program code causing the computing device 130 to execute the method for vascular imaging co-registration as disclosed herein. Additionally, the computer/computing device 130 may be part of or include a system for intravascular imaging registration that includes one or more input port for receiving imaging data; one or more output port; and a controller in communication with the input port and the output port, the controller configured to execute the method for intravascular imaging registration as disclosed herein.

The computer system/sub-system 130 can also include software and hardware that is configured for rendering or displaying imaging, including, for example, extravascular imaging or intravascular imaging derived from the received image data or co-registration method. In some cases, the computer 130 or software can be configured to render both extravascular imaging and intravascular imaging on a single display. In that regard, the system may include a display 150 configured for simultaneously displaying extravascular image data and intravascular image data rendered by the computer 130. The display 150 may be part of the computer system 130 or may be a separate component in communication with the computer system 130, for example through an output port on the computer 130 and a transmission cable 121. In some other cases, however, the communication through the output port may be wireless, rather than by cable. In some examples, the computer 130 or display 150 may be configured to simultaneously provide an angiogram, an IVUS transverse plane view, and an IVUS longitudinal plane view, which may or may not all be co-registered. In other examples, the display may be configured to simultaneously provide an angiogram, an OCT transverse plane view, and an OCT longitudinal plane view, which may or may not be co-registered.

The computer system/sub-system 130 can also include one or more additional output ports for transferring data to other devices. For example, the computer can include an output port to transfer data to a data archive or memory 131. The computer system/sub-system 130 can also include a user interface that may include software and hardware that is configured for allowing an operator to use or interact with the system.

The components of the system 102 may be used cooperatively during a vascular imaging method or procedure that involves the collection of extravascular imaging data and intravascular imaging data during a translation procedure. In the context of performing such a procedure, and obtaining the requisite imaging data, an example method for intravascular imaging registration may be executed or performed.

For example, the patient 100 may be arranged on the table 110 for extravascular imaging of a portion of a blood vessel of interest. The patient 100 or the table may be arranged or adjusted to provide for the desired view of the vessel of interest, in preparation for the collection of extravascular imaging data. Additionally, the intravascular imaging catheter 120 may be introduced intravascularly into the portion of the blood vessel of interest, in preparation for a translation procedure to collect intravascular imaging data. The intravascular imaging catheter 120 can be navigated, and positioned (often under fluoroscopy) within the vessel such that the imaging element is located at a desired starting location for the translation procedure. A guide catheter may be used to aid in navigation. Once in the proper position, a translation procedure may be executed or performed. Before or during the translation procedure, requisite extravascular and intravascular imaging data may be obtained. In this context, or as part of this process, an example method for vascular imaging co-registration or registration may be executed or performed.

### Obtaining Extravascular Imaging Data

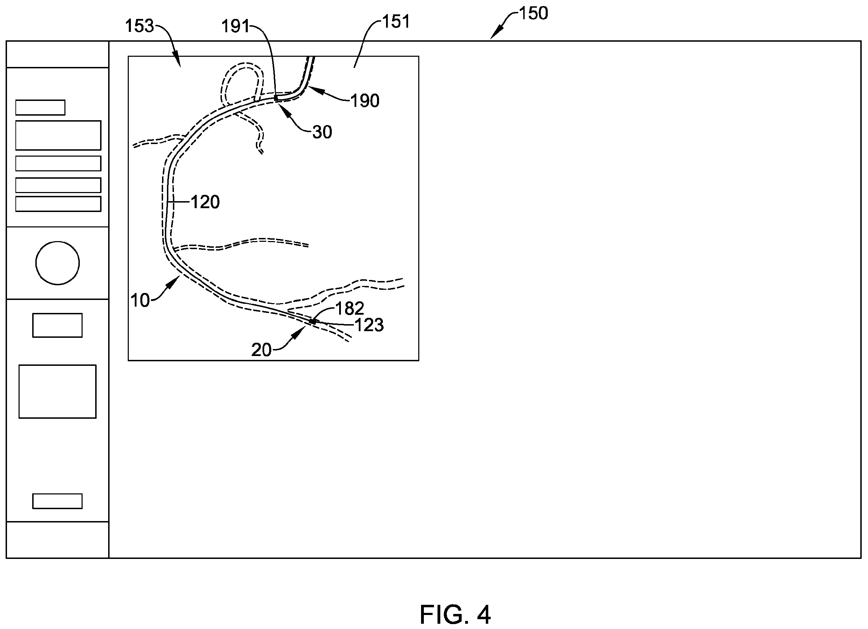

One aspect of the example method for vascular imaging co-registration, includes obtaining extravascular imaging data of a portion of the blood vessel. One component of the obtained extravascular imaging data includes an extravascular image showing the intravascular imaging device disposed within the vessel. This may be represented by FIG. 4, which shows an angiographic/fluoroscopic image 151 generated by the angiography/fluoroscopy system 104 and obtained by the computer 130, and displayed on an image output 153 in the upper left portion of the display 150. The image 151 may be part of a video stream or series of images displayed on the screen of the display 150 at image output 153. The individually rendered frames may be appropriately tagged (e.g., frame number, time stamp, sequence number, etc.) which may be useful to correlate image data frames. As shown, the obtained imaging data can include an extravascular image 151 showing the intravascular imaging device 120 disposed within the vessel 10 (vessel shown in phantom), with an imaging element 182 of the intravascular imaging device 120 disposed at a starting location 20 for the translation procedure. (e.g. sometimes referred to as the "extravascular device image 151"). This extravascular device image 151 may be obtained and recorded under fluoroscopy, through which the radiopaque marker 123 on the device 120 may be used to locate/visualize the location of the imaging element 182 or the starting location 20 of the imaging element 182. Because the starting location 20 has been determined by identifying the actual location from the extravascular image 151, it is an image showing the actual/known location of the intravascular imaging device 120 disposed within the vessel 10 at a particular time, and may be useful during registration. As may be appreciated, the image 151 may be obtained without contrast (e.g. under fluoroscopy) and as such, the anatomy of the vessel 10 may be difficult to discern/visualize (which is why it is shown in phantom).

During the translation procedure, the imaging element 182 will be translated within the blood vessel 10 from the starting location 20 to an ending location 30. This extravascular image 151 may also show the ending location 30 for the translation procedure. For example, as indicated above, a guide catheter 190 including a distal end 191 may be used during the procedure. The distal end 191 may include a radiopaque material or marker, and may be visualized or shown on the obtained extravascular image 151, and used as the ending location for the translation procedure. Because the ending location 30 has been determined by identifying the actual location from the obtained angiographic/fluoroscopic image 151, it is also an actual/known location within the vessel 10, and may also be useful during registration. In other embodiments, other references points shown on the extravascular image 151 may be used to define the starting or ending locations. For example, other devices, stents, anatomical markers, etc., that are shown on the extravascular image 151 may be used.

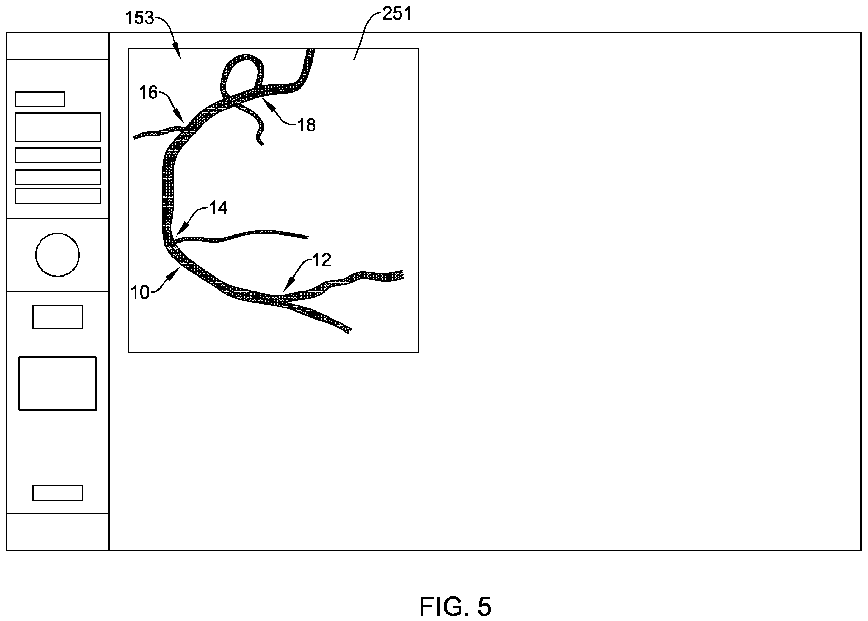

The obtained extravascular imaging data may also include an extravascular contrast image showing the portion of the blood vessel with contrast and showing one or more visualized anatomical landmark(s). This may be represented, for example, by FIG. 5, which shows an extravascular contrast image 251 (e.g. an angiogram taken with contrast media within the vessel 10) generated by the angiography/fluoroscopy system 104 and obtained by the computer 130, and displayed on display 150 at image output 153. The image 251 may be part of the same video stream or series of images as device image 151, and displayed on the screen of the display at image output 153. As shown, the obtained data can include the extravascular contrast image 251 showing the portion of the blood vessel 10 with contrast and showing one or more visualized anatomical landmark(s) (e.g. vessel side branches 12, 14, 16, 18). While in this example, the one or more visualized landmarks include vessel side branches 12, 14, 16, 18, other anatomical landmarks are contemplated and may be shown/used. Some examples of other visualized anatomical landmark that may be shown/used may include: stents, identifiable changes in the size or shape of the vessel 10, such as curves, narrowing, widenings, or the like, or other identifiable anatomical landmark(s) that may be visualized on the extravascular contrast image 251.

The obtained extravascular imaging data may include video data including both the extravascular device image 151 (e.g. image showing the starting position of the intravascular imaging device 120), and the extravascular contrast image 251 (e.g. the "roadmap"). In some cases, the extravascular device image 151 and the extravascular contrast image 251 may be separate individual images that may be combined or superimposed. These images may be obtained automatically by the system as part of a program, which may be initiated by a user, or may be manually requested or obtained by a user interacting with the system, for example, through a user interface.

The order in which the extravascular imaging data is obtained may also vary. For example, as in the shown embodiment of FIGS. 4 and 5, the imaging device 120 may be first navigated to the desired starting location, and the extravascular device image 151 (e.g. FIG. 4) may be obtained first. Thereafter, the extravascular contrast image 251 (e.g. FIG. 5) may be obtained, with the imaging device 120 already in or remaining in the blood vessel 10 when the contrast image 251 is taken. In other embodiments, it is contemplated that the order may be reversed. For example, the extravascular contrast image 251 may be obtained first, without the intravascular imaging device in the blood vessel 10. Thereafter, the imaging device 120 may be navigated to the desired starting location, and the extravascular device image 151 showing the device 120 in the starting location may be obtained. As can be appreciated, the extravascular imaging data may include one or both angiographic image data and fluoroscopic image data. Additionally, as disclosed herein, the extravascular imaging data can be selected from one or more of two-dimensional angiographic image data; three-dimensional angiographic image data; or computer tomography angiographic image data.

### Obtaining Intravascular Imaging Data

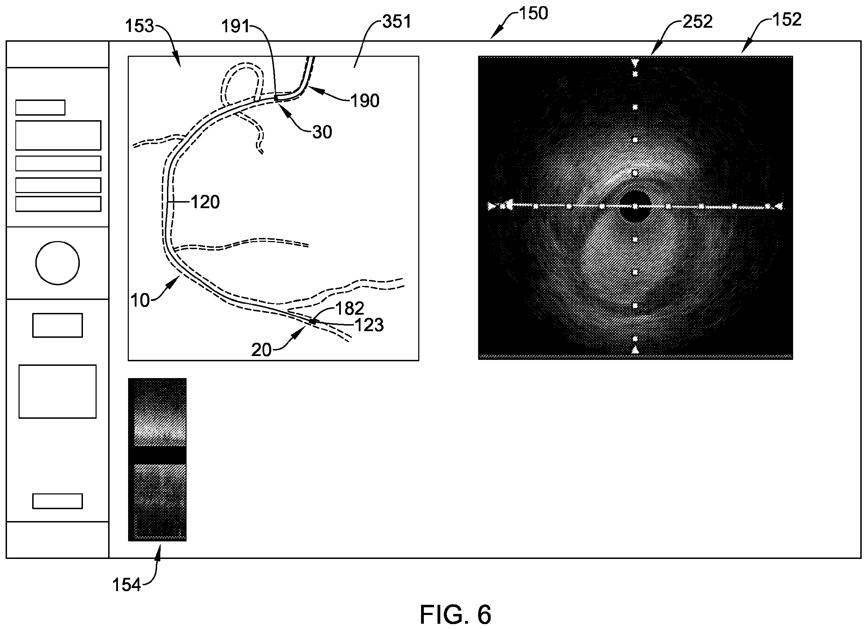

Another aspect of the example method for vascular imaging co-registration includes obtaining intravascular imaging data from the intravascular imaging device during the translation procedure, the intravascular imaging data including one or more intravascular images showing one or more detected anatomical landmark. An example of this may be represented/described with reference to FIGS. 6-8, which is a series of figures which each show intravascular imaging data represented by a video or a series of intravascular images obtained during the progression of the translation procedure. The system may include software that is configured to initiate or perform or facilitate the translation procedure. The translation procedure may be initiated by a user interacting with the system, for example, through a user interface (e.g.: click on a "begin pullback procedure" button to start the translation). Additionally, the translation procedure may be performed using an automated pullback system, for example, at a known speed, as discussed herein.

In this example, the translation procedure is a pullback, where the imaging element 182 is translated within the blood vessel from the starting location 20 to the ending location 30. As may be appreciated, in this example, the intravascular imaging data is intravascular ultrasound data (IVUS). However, as discussed above, in other embodiments, the intravascular imaging data may be generated using other intravascular modalities, for example, optical coherence tomography (OCT), or the like. The obtained intravascular imaging data, in the form of video or a series of images, will include, over the progression of the translation procedure, one or more intravascular images that show one or more detected anatomical landmark. The individually rendered intravascular image frames may be appropriately tagged (e.g., frame number, pullback distance, time stamp, sequence number, etc.) which may be useful to help correlate image data frames, for example, to correlate intravascular image frames and corresponding extravascular (e.g. radiopaque marker) image data frames or to correlate longitudinal cross-sectional intravascular images with transverse cross-sectional intravascular images. As will be seen, in this example, the detected anatomical landmarks that will be detected on IVUS include the four side branches that were shown in the angiogram of this portion of the vessel (e.g. side branches 12, 14, 16, 18 on the angiogram in FIG. 5). However, other detected anatomical landmarks are contemplated and may be shown/used. Some examples of other detected anatomical landmark that may be used include: stents, identifiable changes in the size or shape of the vessel 10, such as curves, narrowing, widenings, or the like, or other detectable anatomical landmark(s) that may be detected with one or more intravascular images obtained during the translation procedure.

Each of FIGS. 6-8 show in the lower portion of the display 150 an image output 154 on the screen including intravascular imaging data including/representing a series of images generated by the intravascular imaging system/sub-system 106, obtained by the computer 130, and displayed on display 150. As can be appreciated, the intravascular images shown in the image output 154 may include a plurality of longitudinal cross-sectional images corresponding to slices of the blood vessel taken along the blood vessel's length during the translation procedure. The intravascular images shown in the image output 154 are obtained during the translation procedure and may be recorded as part of a video or a series of intravascular images. As the translation procedure proceeds, the intravascular imaging data, shown in the image output 154, may be progressively populated with additional intravascular images as they are generated. This is represented, for example, by viewing the progression/lengthening of the image output 154 from FIG. 6 through FIG. 8, where additional intravascular images are progressively being added to the intravascular imaging data 154 on the display 150 as the translation procedure proceeds. One or more of these intravascular images will show one or more detected anatomical landmark(s) (as discussed in further detail herein).

Additionally, each of FIGS. 6-8 show in the upper right portion of the display 150 an image output 152 on the screen including intravascular imaging data including/representing a series of transverse cross-sectional images of vessel segments. These transverse cross-sectional images are included in the imaging data generated by the intravascular imaging system/sub-system 106 and obtained by the computer 130, and displayed at the image output 152 on display 150. These transverse cross-sectional intravascular images shown in the image output 152 are progressively obtained during the translation procedure and may be part of or recorded as an intravascular imaging video or series of intravascular images. At any particular point in time during the translation procedure, the transverse cross-sectional image shown in the image output 152 may correspond to the longitudinal cross-sectional image last added to the intravascular imaging output 154. In other words, the particular transverse cross-sectional image displayed in the image output 152 and the corresponding longitudinal cross-sectional image added at the same time at the end of the image output 154 may be different cross-sectional intravascular images/views, but are taken at the same time/location in the vessel.

During the translation procedure, intermediate extravascular imaging data may also be optionally/periodically obtained, for example, to track the actual progression of the intravascular imaging device 120 during the translation procedure. These intermediate extravascular image(s) may be obtained during the translation procedure, and may show the actual/known location of the intravascular imaging device 120 disposed within the vessel 10 with the imaging element disposed at an intermediate location during the translation procedure, between the starting location 20 the ending location 30. For example, periodically during the translation procedure, the angiography/fluoroscopy system 104 may be activated (e.g. fluoroscopy may be activated) either manually, or automatically, to generate one or more intermediate angiographic/fluoroscopic images, which are obtained by the computer 130, and displayed at that time in the image output 153 on display 150. In at least some embodiments, the translation procedure or the collection of intravascular images or the co-registration method may occur free of continuous angiography/fluoroscopy. In other words, the angiography/fluoroscopy system 104 may be periodically activated, but significant portion or periods of the translation may be performed without active angiography/fluoroscopy. In such embodiments, the actual/known location of the intravascular imaging device 120 is not continuously tracked under angiography/fluoroscopy during the translation procedure.

Each of FIGS. 6-8 show such intermediate/periodically obtained extravascular images in the upper left portion in the image output 153 of the display 150. As can be appreciated, such intermediate extravascular images will show the imaging element 182 disposed at an actual/known intermediate location within the vessel 10, at a particular time during the translation procedure. At the particular point in time during the translation procedure when the intermediate extravascular image is generated and shown in the image output 153 on display 150, the corresponding longitudinal cross-sectional image for that particular intermediate location within the vessel will be shown as the last image added in the intravascular imaging output 153. Likewise, the transverse cross-sectional image shown in the image output 152 at that time will also correspond to the particular intermediate location within the vessel 10.

Briefly running through the progression of FIGS. 6-8, FIG. 6, shows intravascular images taken near the beginning of the translation procedure. Because it is early in the translation procedure, the image output 154 on the bottom of the screen is relatively short, showing the relatively few longitudinal cross-sectional intravascular images obtained thus far in the translation procedure. In the upper right portion of the display 150, image output 152 shows a corresponding intravascular transverse cross-sectional image 252 of vessel at this location. FIG. 6 also shows an intermediate extravascular imagine 351 in the image output 153 on display 150. The intermediate extravascular imagine 351 may be obtained by brief activation of the fluoroscope to discern the actual/known location of the intravascular imaging device 120 at this time in the translation procedure, which in this instance, shows the imaging element disposed closer to the starting location 20 than to the ending location 30.

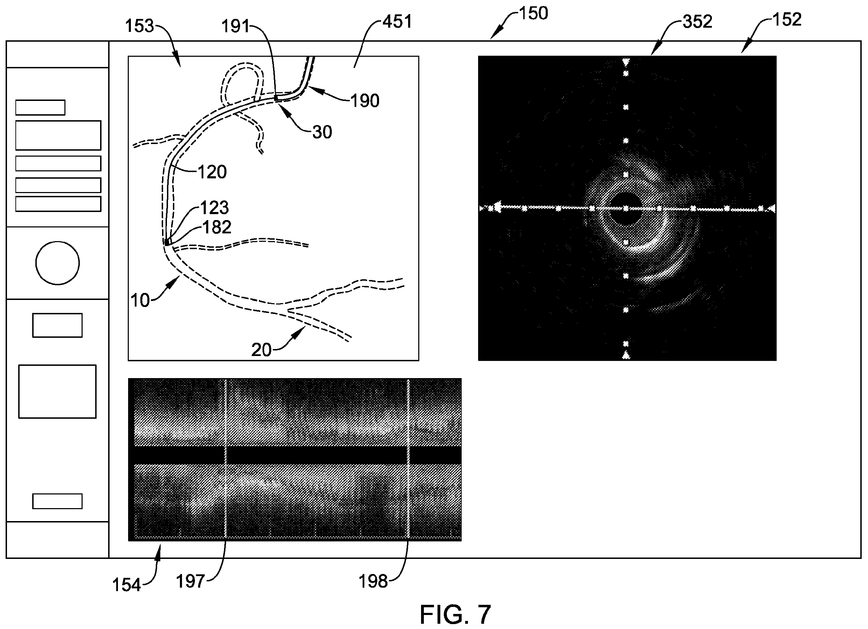

FIG. 7 shows intravascular images taken near the middle of the translation procedure. The image output 154 on the bottom of the screen is growing (as compared to FIG. 6), showing the addition of more longitudinal cross-sectional intravascular images obtained as the translation procedure progresses. It may also be appreciated that one or more of these intravascular images will show one or more detected anatomical landmark(s). In this case, the detected anatomical landmarks are detected side branches of the vessel 10. In FIG. 7, these intravascular images showing the first two side branches (e.g. detected anatomical landmarks) are marked with lines 197 and 198. In some cases, the detected anatomical landmark may be detected or marked automatically by the system. For example, the computer 130 may include software or hardware that is configured to perform image processing and image-recognition. Using the intravascular image data (e.g. IVUS data or other suitable data), the system may perform image processing and image-recognition to identify or mark the images including the detected anatomical landmarks 197/198. In other cases, the images including the detected anatomical landmarks may be identified or marked manually by a user, for example, through a user interface.

In the upper right portion of the display 150 of FIG. 7, the image output 152 shows a corresponding intravascular transverse cross-sectional image 352 of vessel at this location. FIG. 7 also shows a second intermediate extravascular imagine 451 in the image output 153 on display 150. The intermediate extravascular imagine 451 may be obtained by brief activation of the fluoroscope to discern the actual/known location of the intravascular imaging device 120 at this time in the translation procedure, which in this instance, shows the imaging element disposed about half way between the starting location 20 and the ending location 30.

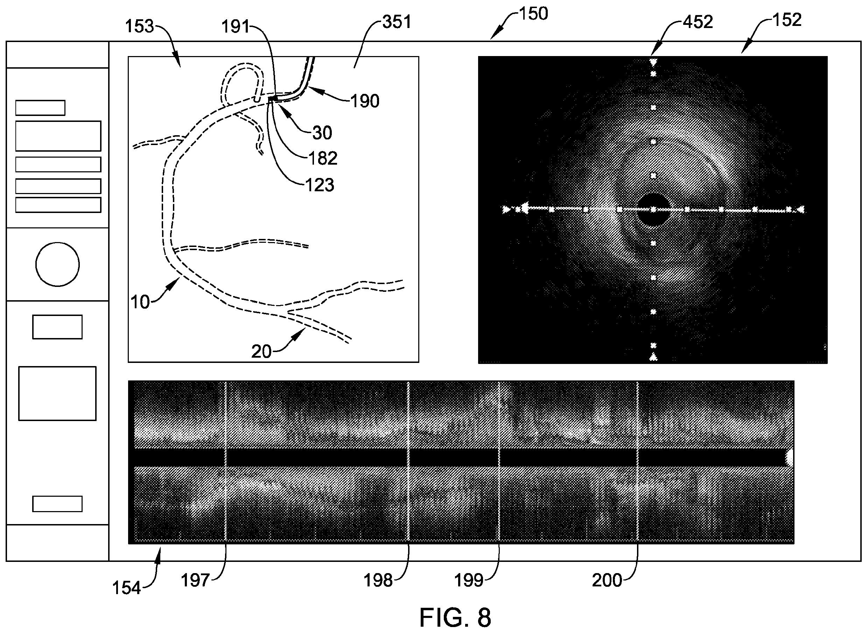

FIG. 8 shows intravascular images taken as the translation procedure is approaching an end or has ended. The image output 154 on the bottom of the screen has grown across the display (as compared to FIGS. 6 and 7), showing the addition of yet more longitudinal cross-sectional intravascular images obtained through the complete translation procedure (from start to end). It may also be appreciated that one or more of these intravascular images in the image output 154 on display 150 will show one or more detected anatomical landmark(s) (e.g. detected side branches of the vessel). In FIG. 8, intravascular images showing the first two detected side branches are again marked with lines 197 and 198 (as in FIG. 7). Two additional intravascular images showing a third and fourth detected side branches are now also obtained during the latter half of the translation procedure, and are marked with lines 199 and 200. As discussed herein, the images including the detected anatomical landmark may be identified or marked automatically, for example, using image processing and image-recognition, or may be identified or marked manually by a user, for example, through a user interface.

In the upper right portion of the display 150, image output 152 shows a corresponding intravascular transverse cross-sectional image 452 of vessel that corresponds to the ending location in the vessel. FIG. 8 also shows an additional (e.g. third) intermediate extravascular imagine 551 in the image output 153 on display 150. The extravascular image 551 may be obtained by brief activation of the fluoroscope to discern the actual/known location of the intravascular imaging device 120, which at the end of the translation procedure, is shown with the imaging element 182 disposed at the ending location 30. At this point, translation procedure can be stopped, as the imaging element 182 has reached the ending location 30. This may be done automatically by the system, or may be done manually by a user interacting with the system.

### Marking one or more Known Location on the Extravascular Imaging Data

Another aspect of the example method for vascular imaging co-registration includes marking the extravascular imaging data for registration. For example, the extravascular imaging data may be marked with actual/known registration points. For example, some embodiments involve marking the starting location 20 and/or the ending location 30 of the translation procedure on the extravascular imaging data.

For example, as described herein, the obtained extravascular imaging data includes an extravascular image 151 (e.g. FIG. 4) and also an extravascular contrast image 251 (e.g. FIG. 5). The extravascular image 151 was obtained under fluoroscopy, and shows the starting location 20 and/or the ending location 30 for a translation procedure, which were discerned and identified using positions or radiopaque markers of the devices 120/190 identified under fluoroscopy. These are examples of actual/known locations or registration points. However, the vessel lumen or anatomical landmarks, such as side branches, may not be readily discernable/identifiable in image 151, due to the absence of contrast flow. (but they are shown in phantom in FIG. 4 for reference). The extravascular contrast image 251 (FIG. 5), on the other hand, is an angiogram with contrast, thus showing the portion of the blood vessel with contrast and showing one or more visualized anatomical landmark(s). The visualized anatomical landmarks (e.g. side branches) can thus be identified on the extravascular contrast image 251. However, due to the contrast, the position of the devices or radiopaque markers of the devices 120/190 are less discernable or not identifiable in the extravascular contrast image 251. This makes it difficult or impossible to see or identify, for example, actual/known locations or registration points, such as the starting location 20 and the ending location 30 for a translation procedure. In some embodiments, it may be desirable to provide an image for marking or registration that includes data from both an extravascular image without contrast (e.g. fluoroscopy image 151) and an extravascular contrast image (e.g. contrast image 251).

In some embodiments, the data from the extravascular image 151, showing actual/known locations or registration points, such as the starting location 20 and the ending location 30 for a translation procedure, may be combined with or superimposed onto the extravascular contrast image 251. In some cases, this process may be done automatically by the system. For example, the computer 130 may include software or hardware that is configured to perform image processing and image-recognition designed to combined or superimpose the data in images. In other cases, the images may be combined or superimposed manually by a user, for example, through a user interface.

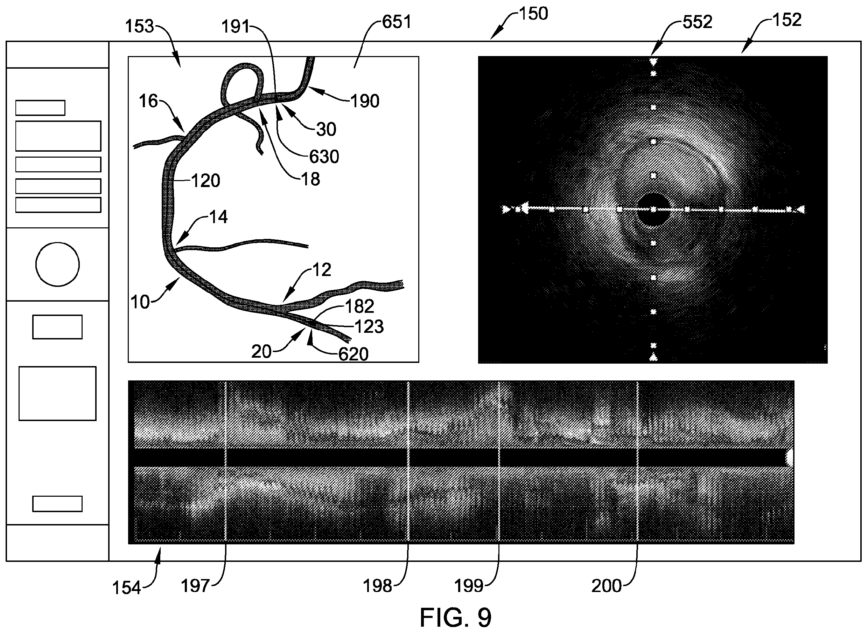

The result of combining or overlaying/superimposing the data from extravascular image 151 (e.g. fluoroscopy image) with the data from extravascular contrast image 251 (e.g. angiogram with contrast) may result in a combined or enhanced extravascular image 651 (e.g. enhanced angiogram), which is extravascular imaging data that is or can be marked. One example of such an extravascular image 651 is depicted in FIG. 9, which shows an extravascular image 651 generated by the computer 130, and displayed on the image output 153 in the upper left portion of the display 150.

As can be appreciated in FIG. 9, the starting location 20 or the ending location 30 (e.g. actual/known locations or registration points) are identified on the extravascular image 651 and may be marked on the extravascular imaging data. For example, the starting location 20 may be marked with a marker 620 on the extravascular image 651. The ending location 30 may be marked with a marker 630 on the extravascular image 651. In some cases, this identification or marking process may be done automatically by the system. For example, the computer 130 may include software or hardware that is configured to perform image processing and image-recognition configured to identify and mark the starting location 20 or the ending location 30 (or other actual/known locations or registration points). In other cases, marking the starting location 20 or the ending location 30 (or other actual/known locations or registration points) be done manually by a user, for example, through a user interface.

Other extravascular data or images including or showing other actual/known locations of the imaging element of the intravascular imaging device 120 during the translation procedure may also be combined with or superimposed onto the extravascular contrast image 251 or the extravascular image 651. For example, other extravascular images, such as intermediate extravascular image 351 (FIG. 6) or intermediate extravascular image 451 (FIG. 7) or intermediate extravascular image 551 (FIG. 8) were also each obtained under fluoroscopy during the translation procedure, with each including an actual/known location of the imaging element 182 (due to radiopaque marker 123) of the intravascular imaging device 120 at a certain point during the translation procedure. Combining or superimposing the data from one or more of these additional extravascular images with the contrast image 251 or the extravascular image 651 may add extra reference points, and may help to enhance accuracy. The process of combining or superimposing the data may be done as discussed above, for example, automatically by the system or manually by a user. Additionally, the particular actual/known location(s) of the imaging element 182 may also be marked on the extravascular imaging data, with the marking occurring similar to as discussed above.

FIG. 9 also shows intravascular images taken as the translation procedure is approaching an end or has ended. The image output 154 on the bottom of the screen shows longitudinal cross-sectional intravascular images obtained during the translation procedure (from start to end). The image output 154 includes intravascular images showing the four detected anatomical landmarks (e.g. side branches) which are again marked with lines 197, 198, 199 and 200 respectively. In the upper right portion of the display 150, image output 152 shows a corresponding intravascular transverse cross-sectional image 552 of vessel that corresponds to the ending location in the vessel.

As discussed herein, at least parts of the translation procedure during the collection of intravascular images may occur or be performed free of continuous angiography/fluoroscopy. In other words, the angiography/fluoroscopy system 104 may not be, or only periodically be activated during the translation procedure, for example to obtain actual/known locations. But significant portion or periods may be performed without angiography/fluoroscopy. In such cases, the actual/known location of the intravascular imaging device 120 is not continuously tracked under angiography/fluoroscopy during the translation procedure.

In embodiments where angiography/fluoroscopy is inactive during significant portions of the translation procedure, the system may be configured to calculate an approximate or predicted location of the imaging element 182 (e.g. due to the radiopaque marker 123) for those portions of the translation procedure when the angiography/fluoroscopy is inactive. For example, such calculations may be based upon its last registered position/location (e.g. last registered actual/known location of the imaging element 182) and other indicators of catheter movement or location, such as a known pullback distance and speed, a calculated path, or other non-visual position data, or the like, etc.

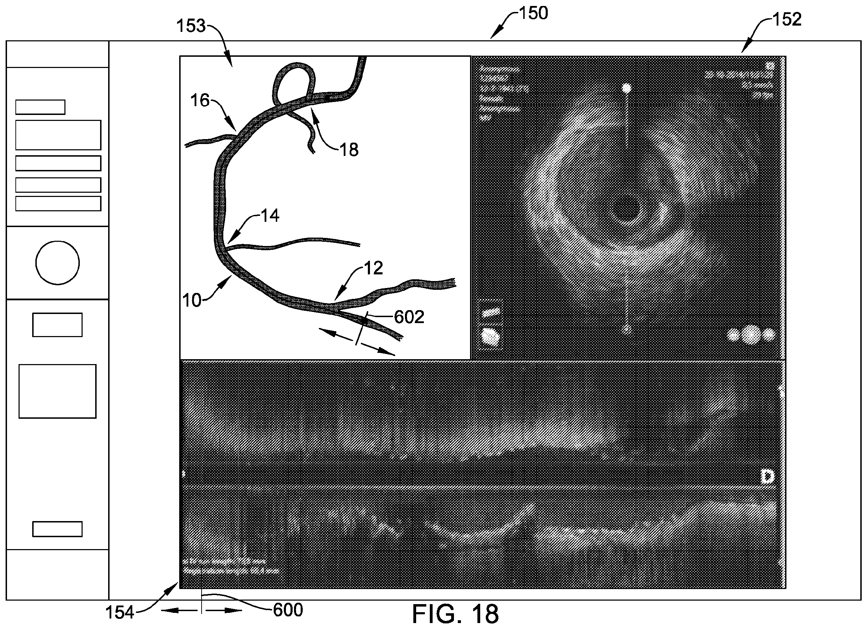

For example, if an initial location of the imaging element 182 is known (e.g. through one or more actual/known locations or registration points - such as the starting location 20, ending location 30, or one of the intermediate locations obtained during the translation procedure, etc., - and the catheter 120 is pulled by an automatic pullback system at a specific rate for a known amount of time, the calculated/predicted location will be a distance from the initial location along the path of travel, and is represented by the product of the pullback rate and the time period. The computer 130, or components thereof, can include software or hardware designed to make such calculations, and output the results, for example showing or marking the calculated/predicted location on displayed images, as desired. For example, a calculated/predicted location for a particular point during the translation procedure may be superimposed upon the extravascular image 651 or a co-registered image such as that shown in FIG. 18, and may represent the calculated/predicted location of the probe 122 or imaging element 182 at that point in the translation procedure.

In some embodiments, a calculated path that the imaging element 182 takes (e.g. calculated path of travel) during the translation procedure may be determined or used or displayed. For example, a predicted/calculated path may extend between the starting location 20 and the ending location 30, and may generally extend along the imaged vessel lumen shown on extravascular contrast imaging data. Data regarding the calculated path may also be used or considered when calculating an approximate or predicted location of the imaging element 182. Some examples of methods that may be used to determine a calculated path include: user-specified points or manual path specification; image pattern recognition; automated two-dimensional and three-dimensional path calculations; user assisted automated path calculations; and combinations of manual and automated calculations of a path. The computer 130, or the components thereof, can include software or hardware designed to make or facilitate such calculations, and output the results, for example showing or marking the calculated path on displayed images, as desired. For example, the calculated path may be superimposed upon the extravascular image 651 or a co-registered image such as that shown in FIG. 18, and may represent the projected path of the probe 122 or imaging element 182 during the translation procedure.

As may be appreciated, there may be error between the calculated/predicted location and the actual/known location. For example, it is expected that at certain periods during which fluoroscopy is inactive, foreshortening issues may be present and cause error between the calculated/predicted location and the actual/known location, especially in a tortuous/winding vessel. However, each subsequent time that the fluoroscope is activated and actual/known location data is acquired and presented to the processor, error between the actual/known location and the predicted/calculated location may be reduced or eliminated by replacing the calculated/predicted position with the actual/known location. Additionally, another aspect of the example method for vascular imaging co-registration disclosed herein includes aligning the predicted location of a particular detected anatomical landmark with a corresponding visualized anatomical landmark, as will be discussed in more detail below. This aspect may also help to alleviate or reduce error/misalignment.

### Marking a Predicted Location of a Detected Anatomical Landmark on the Extravascular Imaging Data

Another aspect of the example method for vascular imaging co-registration includes marking a predicted location of detected anatomical landmark(s) on the extravascular imaging data. The intravascular imaging data obtained during the translation procedure will include one or more intravascular images showing one or more detected anatomical landmarks. As can be appreciated, these intravascular images showing the detected anatomical landmarks are included in the intravascular imaging data, obtained using the intravascular imaging device during the translation procedure. For co-registration purposes, the location of these detected anatomical landmark (e.g. from IVUS or OCT data) will be correspondingly identified and/or marked and/or registered on the extravascular imaging data (e.g. the angiography/fluoroscopy data). It is useful to know the location (either actual/known location or calculated/predicted location) of the imaging element 182 of the intravascular imaging device 120 when it detected a particular detected anatomical landmark during the translation procedure, which can then be used to mark and/or register that location (of the detected anatomical landmark) on the extravascular imaging data (e.g. the angiography/fluoroscopy data).

In certain specific situations, where it happens that the extravascular imaging device (angiography/fluoroscopy) is active at the same time that the imaging element 182 detects a particular detected anatomical landmark, then the location of the detected anatomical landmark is actual/known. The location of that particular detected anatomical landmark can be marked and/or registered at that location on the extravascular imaging data, using the actual/known location provided by the extravascular imaging.

However, as discussed herein, at least portions of, if not the majority or all of, the translation procedure when intravascular images are collected are performed free of continuous extravascular imaging (e.g. free of angiography/fluoroscopy). In such cases, the actual/known location of the imaging element 182 on the intravascular imaging device 120 as it detects a particular detected anatomical landmark during the translation procedure will not be known. As such, a calculated/predicted location of the imaging element 182 on intravascular imaging device 120 as it detects a particular detected anatomical landmark during the translation procedure will be used. Methods and/or systems for determining the calculated/predicted location of the imaging element 182 are described above, and may be used in this context. The predicted location of detected anatomical landmark(s) are then marked on the extravascular imaging data.

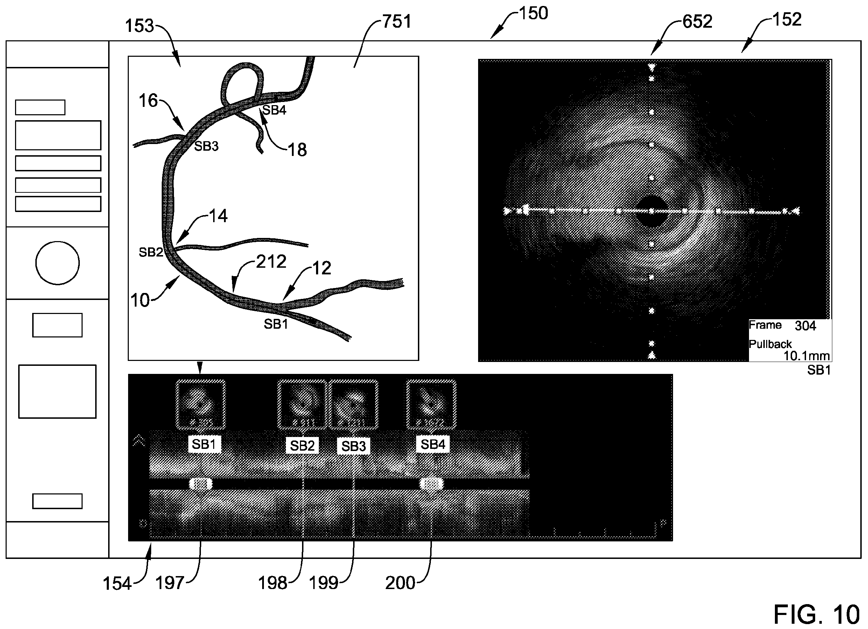

For example, FIG. 10 shows extravascular imaging data, in this case an extravascular image 751, which may be created in a similar manner, and be similar in form and function to the extravascular image 651 of FIG. 9. The extravascular image 751 shows the visualized anatomical landmarks, in this case, side branches 12, 14, 16, and 18. Additionally, the predicted location of detected anatomical landmark 197 (e.g. the first side branch) from the intravascular images 154 has been calculated/predicted (as discussed herein). The predicted location of the detected anatomical landmark 197 is then marked on the extravascular imaging data (e.g. the extravascular image 751) with marker arrow 212. As can be appreciated, in this instance, there is some misalignment/error/discrepancy between the predicted and marked location 212 for the first side branch, and the visualized anatomical landmark 12 on the extravascular imaging data.

It is also noted that in the extravascular image 751, the visualized anatomical landmarks (e.g. side branches) are identified and/or identifiable - either manually or by the system. In some embodiments, the visualized anatomical landmarks (e.g. side branches 12, 14, 16, 18) may simply be identified manually by a user, for example, by the user evaluating the image on the screen. In some embodiments, the visualized anatomical landmarks (e.g. side branches 12, 14, 16, 18) may be identified automatically by the system. For example, the computer 130 may include software or hardware that is configured to perform image processing and image-recognition. Using the extravascular image data (e.g. angiographic data), the system may perform image processing and image-recognition to identify the visualized anatomical landmarks (e.g. side branches 12, 14, 16, 18).

The visualized anatomical landmarks may also be marked on the extravascular imaging data. For example, the visualized anatomical landmarks (e.g. side branches 12, 14, 16, 18) may be marked with appropriate markers and/or labels on the extravascular image 751. In this case, side branch 12 is marked as SB1, side branch 14 is marked as SB2, side branch 16 is marked as SB3, and side branch 18 is marked as SB4. In some cases, this identification or marking process may be done automatically by the system. For example, the computer 130 may include software or hardware that is configured to perform image processing and image-recognition configured to identify and mark the visualized anatomical landmarks. In other cases, marking the visualized anatomical landmarks may be done manually by a user, for example, through a user interface.

FIG. 10 also shows intravascular images taken during the translation procedure. The image output 154 on the bottom of the screen shows longitudinal cross-sectional intravascular images obtained during the translation procedure (from start to end). The image output 154 includes intravascular images showing the four detected anatomical landmarks (e.g. side branches) which are again identified and then marked with lines 197, 198, 199 and 200, as elsewhere herein. It may also be appreciated that lines 197, 198, 199 and 200 now bear labels and/or flags. In particular, lines 197 now bears label SB1, line 198 now bear label SB2, line 199 now bears label SB3, and line 200 now bears label SB4. The markings/text/symbols on the labels denoting the four detected anatomical landmarks (e.g. side branches detected on IVUS) correspond with the markings/text/symbols on the labels given the corresponding visualized anatomical landmarks (e.g. side branches detected on the angiogram). Additionally, each of the detected anatomical landmarks now also bear an additional label showing a small representation of the corresponding intravascular transverse cross-sectional image for that particular detected anatomical landmark. These small images of the corresponding intravascular transverse cross-sectional images can be seen above each text labels for each detected anatomical landmark. In some cases, these marking/labels may be applied automatically by the system. For example, the computer 130 may include software or hardware that is configured to perform image processing and image-recognition configured to identify and marking/labels the landmarks accordingly. In other cases, marking/labeling may be done manually by a user, for example, through a user interface.

In some cases, these lines/markings/labels may be interactive. For example, through a user interface, a user may actuate one of the lines/markings/labels, and the lines/markings/labels may become highlighted and/or activated. When a line/marking/label for a particular detected anatomical landmark is highlighted or activated, the corresponding marked predicted location of for that particular detected anatomical landmark is shown on the extravascular imaging data (e.g. the extravascular image 751), and the corresponding intravascular transverse cross-sectional image 652 for that particular detected anatomical landmark is shown in the image output 152 in the upper right portion of the display 150.

For example, as seen in FIG. 10, the label SB1 (designating detected anatomical landmark 197) is shown being activated/highlighted, as is represented by the downward arrow above this label. Marker arrow 212, designating the predicted location of the detected anatomical landmark 197 on the extravascular imaging data is then shown on the extravascular image 751. And the corresponding intravascular transverse cross-sectional image for detected anatomical landmark 197 is shown in the image output 152. Each of the other labels may be similarly activated to show corresponding information.

### Aligning the Predicted Location of the Detected Anatomical Landmark with the Visualized Anatomical Landmark

Another aspect of the example method for vascular imaging co-registration includes aligning the predicted location of the detected anatomical landmark with the visualized anatomical landmark.

As discussed herein, the visualized anatomical landmark(s) are identified and/or identifiable on the extravascular imaging data - either manually or automatically by the system. The visualized anatomical landmarks may also, optionally, be marked on the extravascular imaging data - either manually or by the system. Further, the predicted location(s) of detected anatomical landmark(s) may be calculated and marked on the extravascular imaging data - either manually or automatically by the system. However, as discussed herein, there may be some misalignment/error/discrepancy between the predicted and marked location(s) of detected anatomical landmark(s) on the extravascular imaging data, and the corresponding visualized anatomical landmark on the extravascular imaging data. The disclosed method for vascular imaging co-registration may include aligning the predicted location of the detected anatomical landmark with the visualized anatomical landmark, and may help to alleviate this misalignment/error/discrepancy.

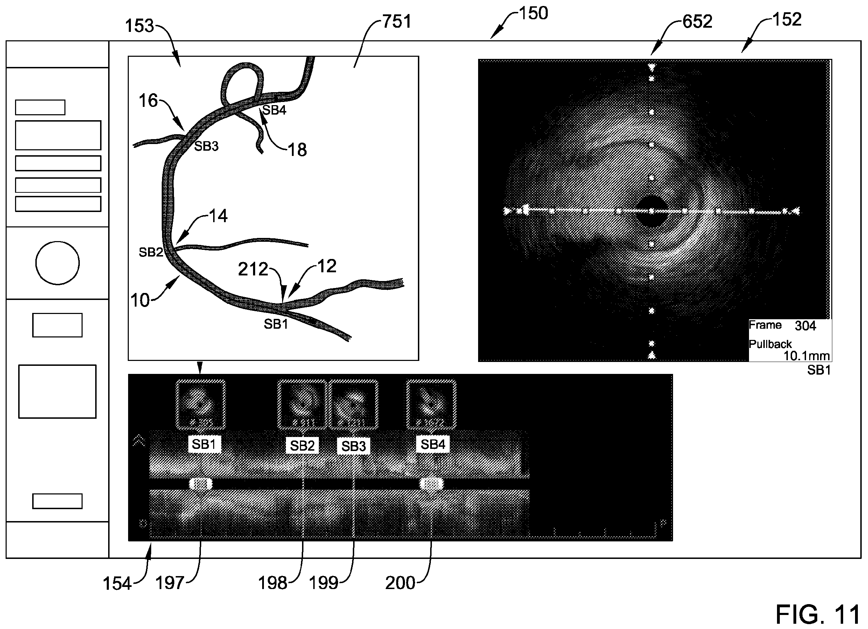

For example, FIG. 10 shows the display including extravascular imaging data, in this case an extravascular image 751, which shows the visualized anatomical landmark 12. Landmark 12 is identified and labeled SB1 (e.g. side branch 1) on the extravascular image 751. Additionally, the predicted location of detected anatomical landmark 197 (also labeled SB1) from the intravascular images 154 was calculated/predicted and is marked with marker arrow 212 on the extravascular image 751. As can be appreciated, there is some misalignment/error/discrepancy between the predicted location 212 for the detected anatomical landmark, and the identified visualized anatomical landmark 12 on the extravascular imaging data. The method and system disclosed herein provides for aligning the predicted location 212 with the visualized anatomical landmark 12. The alignment is typically done by moving or dragging the indicator representing the predicted location 212 on the screen such that it aligns with the visualized anatomical landmark 12. In some cases, this alignment process may be done automatically by the system. For example, the computer 130 may include software or hardware that is configured to automatically align the predicted location 212 with the corresponding visualized anatomical landmark 12. This may be the done at the instruction of a user, or may be done automatically if/when misalignment is detected. In other cases, the alignment may be performed manually by a user, for example, through a user interface. Some examples of manual alignment may include the use of the interactive labels discussed above. For example, through a user interface, a user may highlight/actuate the label corresponding to the detected anatomical landmark of interest - in this case detected anatomical landmark 197 bearing label SB1. When the label for detected anatomical landmark is highlighted/activated, the corresponding marked predicted location of for that particular detected anatomical landmark is then shown on the extravascular imaging data - in this case marker arrow 212, designating the predicted location of the detected anatomical landmark 197. The user may then manually move/drag the marker arrow 212 into alignment with the corresponding visualized anatomical landmark - in this case, visualized anatomical landmark 12. The user may then inactivate the label, for example to thereby saving the alignment. FIG. 11 shows a screen with same imaging data as shown in FIG. 10, but after aligning the predicted location 212 with the visualized anatomical landmark 12.

A similar alignment steps may also be done for any other predicted locations of additional detected anatomical landmark and corresponding visualized anatomical landmarks that are misaligned.

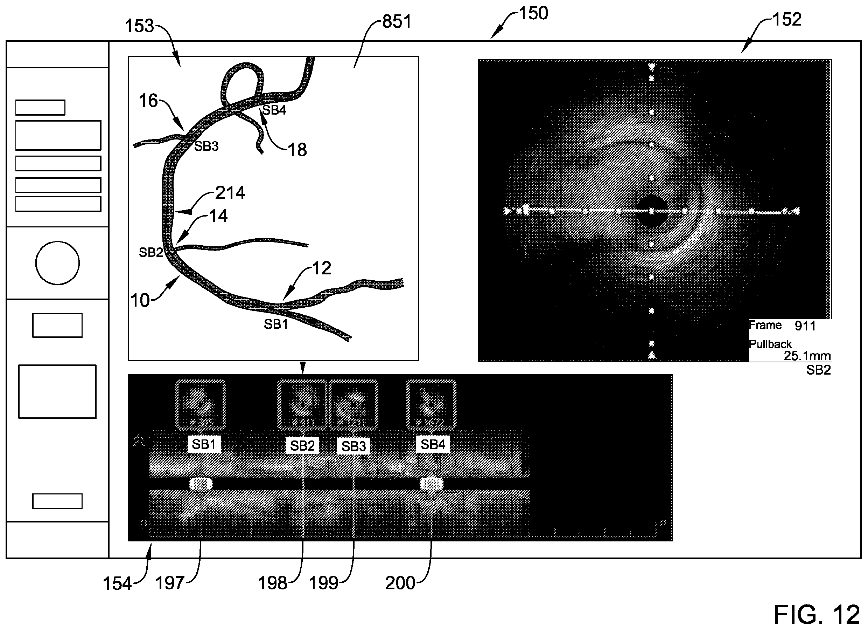

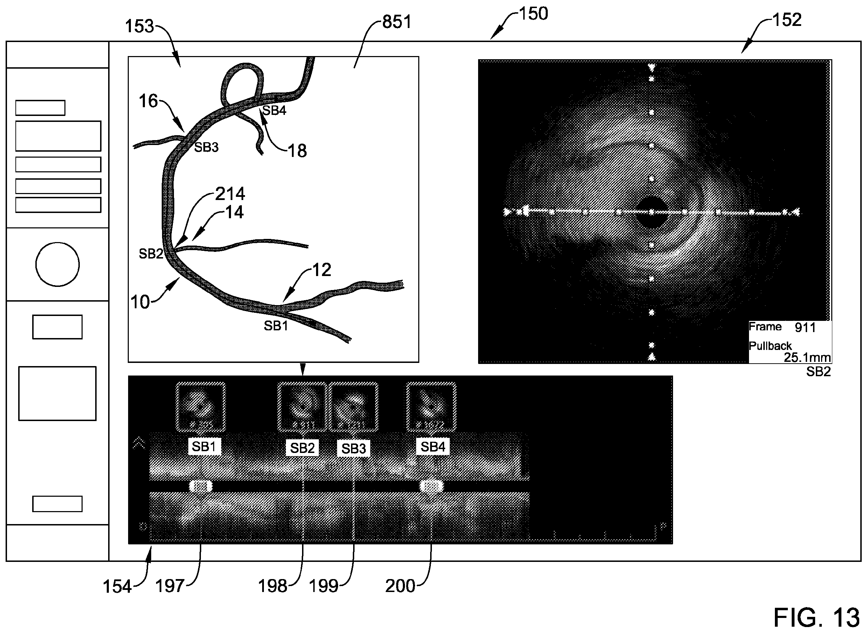

For example, FIG. 12 shows the display including extravascular imaging data, in this case an extravascular image 851 which is basically the same as extravascular image 751, but with a focus on visualized anatomical landmark 14 and the corresponding predicted location of detected anatomical landmark 198. Landmark 14 is identified and labeled SB2 (e.g. side branch 2) on the extravascular image 851. Additionally, the predicted location of detected anatomical landmark 198 (also labeled SB2) from the intravascular images 154 was calculated/predicted and is marked with marker arrow 214 on the extravascular imaging image 851. As can be appreciated, there is some misalignment/error/discrepancy between the predicted location 214 for the detected anatomical landmark, and the identified visualized anatomical landmark 14 on the extravascular imaging data. The method and system disclosed herein also provides for aligning the predicted location 214 with the visualized anatomical landmark 14 in a similar manner as discussed above for aligning the predicted location 212 with the visualized anatomical landmark 12. FIG. 13 shows a screen with same imaging data as shown in FIG. 12, but after aligning the predicted location 214 with the visualized anatomical landmark 14.

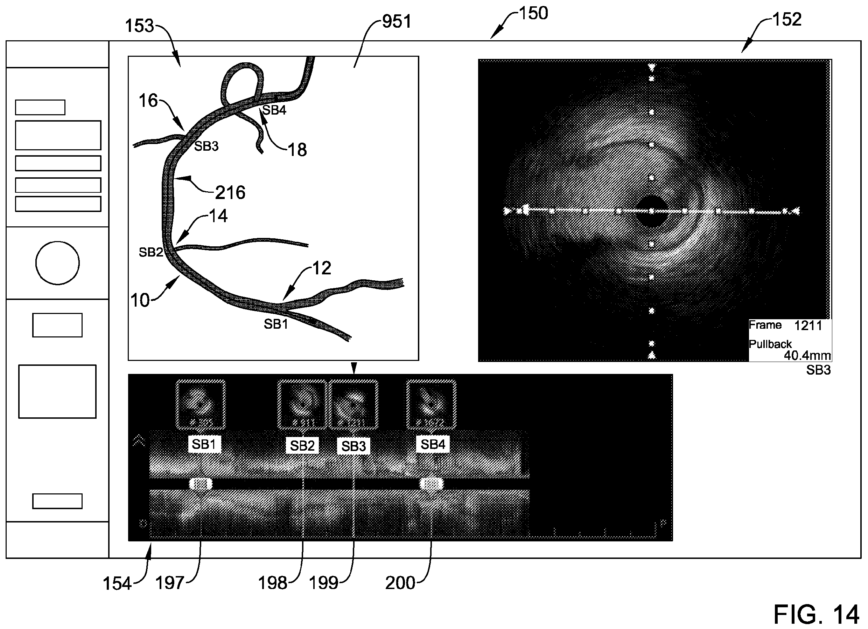

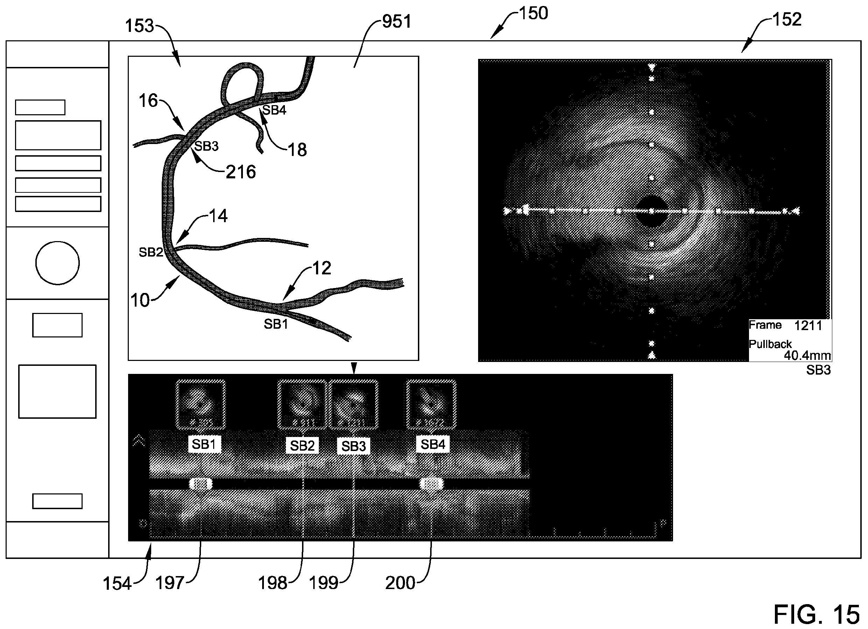

Similarly, FIG. 14 shows the display including extravascular imaging data, in this case an extravascular image 951 which is basically the same as extravascular images 751 and 851, but with a focus on visualized anatomical landmark 16 and the corresponding predicted location of detected anatomical landmark 199. Landmark 16 is identified and labeled SB3 (e.g. side branch 3) on the extravascular image 951. Additionally, the predicted location of detected anatomical landmark 199 (also labeled SB3) from the intravascular images 154 was calculated/predicted and is marked with marker arrow 216 on the extravascular imaging image 951. As can be appreciated, there is some misalignment/error/discrepancy between the predicted location 216 for the detected anatomical landmark, and the identified visualized anatomical landmark 16 on the extravascular imaging data. The method and system disclosed herein also provides for aligning the predicted location 216 with the visualized anatomical landmark 16 in a similar manner as discussed above for aligning the predicted location 212 with the visualized anatomical landmark 12. FIG. 15 shows a screen with same imaging data as shown in FIG. 14, but after aligning the predicted location 216 with the visualized anatomical landmark 16.

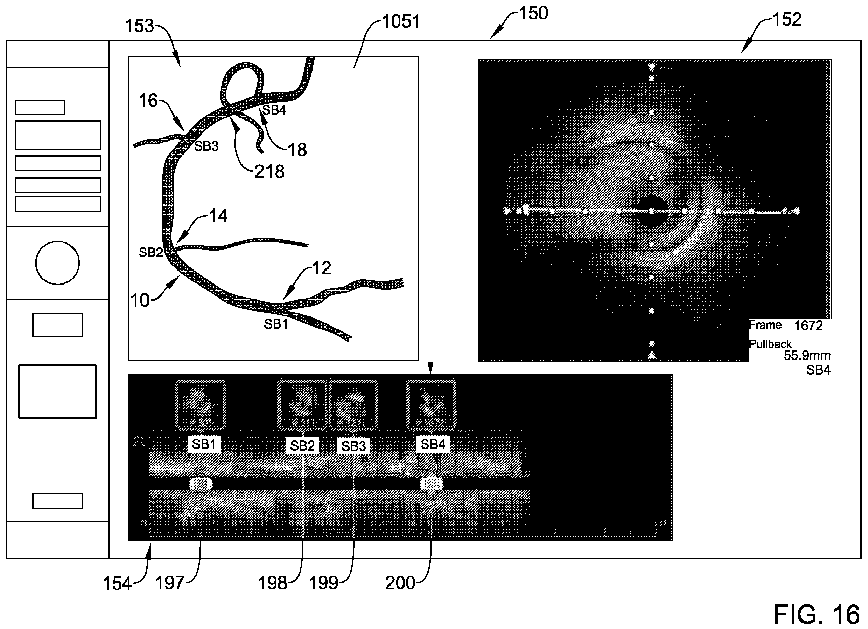

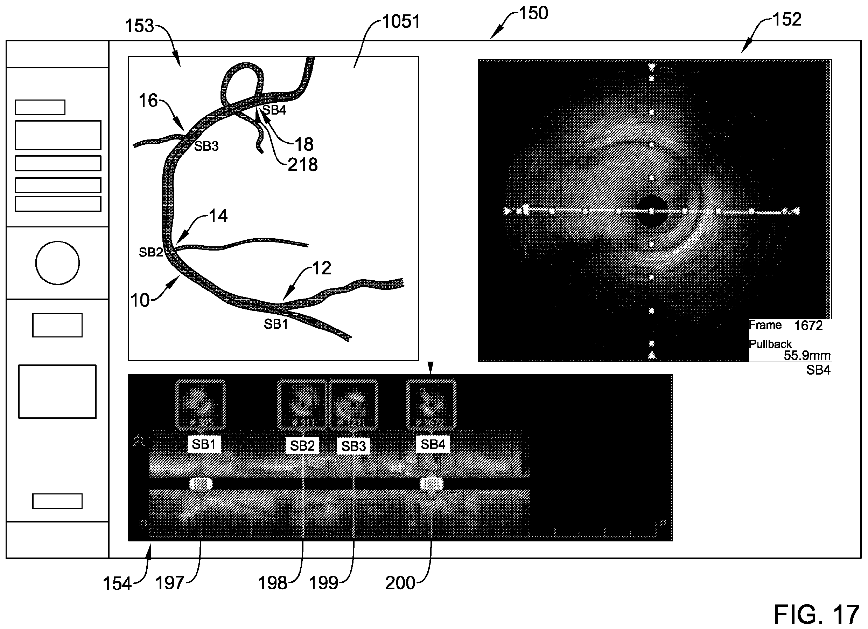

Similarly, FIG. 16 shows the display including extravascular imaging data, in this case an extravascular image 1051 which is basically the same as extravascular images 751, 851 and 951, but with a focus on visualized anatomical landmark 18 and the corresponding predicted location of detected anatomical landmark 200. Landmark 18 is identified and labeled SB4 (e.g. side branch 4) on the extravascular image 1051. Additionally, the predicted location of detected anatomical landmark 200 (also labeled SB4) from the intravascular images 154 was calculated/predicted and is marked with marker arrow 218 on the extravascular imaging image 1051. As can be appreciated, there is some misalignment/error/discrepancy between the predicted location 218 for the detected anatomical landmark, and the identified visualized anatomical landmark 18 on the extravascular imaging data. The method and system disclosed herein also provides for aligning the predicted location 218 with the visualized anatomical landmark 18 in a similar manner as discussed above for aligning the predicted location 212 with the visualized anatomical landmark 12. FIG. 17 shows a screen with same imaging data as shown in FIG. 16, but after aligning the predicted location 218 with the visualized anatomical landmark 18.

As a result of the describes methods and/or systems as described herein, the intravascular images generated by the intravascular catheter during the translation procedure are co-registered to the extravascular images, so that every intravascular image (e.g. IVUS or OCT image) is linked to its corresponding location on the extravascular image (e.g. an angiographic image). The resulting co-registered images can be used to render and present a co-registered display including both the extravascular images and the corresponding intravascular images. The co-registered extravascular and intravascular images can be simultaneously displayed, along-side one another, upon the display 150. The co-registered image data may also be stored in the computer 130 or in a long-term storage device 131 for later review, for example in a session separate from the procedure that acquired the extravascular and intravascular image data. The co-registered display may also be rendered in playback mode.

FIG. 18 shows an example of such a resulting co-registered display including both the extravascular images and the corresponding intravascular images. As can be appreciated, the co-registered extravascular and intravascular images can be simultaneously displayed, along-side one another, upon the display 150. For example, the display 150 includes image output 153 in the upper left portion for showing co-registered extravascular imaging data, image output 154 along the bottom for showing co-registered longitudinal cross-sectional intravascular images obtained during the translation procedure (e.g. from start to end), and image output 152 in the upper right portion for showing corresponding co-registered transverse cross-sectional intravascular images obtained during the translation procedure.

In some embodiments, the system may include software or hardware that is configured to allow a user to scroll and/or track through the series of co-registered images. For example, the system may include a configuration that allows a user to scroll through one of the sets of co-registered images, and as the scrolling occurs, the processor acquires and displays corresponding co-registered images for the other sets of images. For example, with reference to FIG. 18, a slider bar/cursor 600 may be displayed/overlaid on the image output 154, which shows the series of intravascular images obtained during the translation procedure. Additionally, corresponding slider bar/cursor 602 may be displayed on the image output 153 along the extravascular image of the vessel. The two bar/cursors 600/602 may be associated and/or linked, such that when a user selects and drags the slider bar/cursor 602 along the path of the vessel (e.g. a calculated path) on the co-registered extravascular image shown in the image output 153, the bar/cursor 600 correspondingly slides along the corresponding/co-registered images shown in the image output 154. Similarly, when a user selects and drags the slider bar/cursor 600 along the co-registered images shown in the image output 154, the bar/cursor 602 slides along the path of the vessel (e.g. a calculated path) on the co-registered extravascular image shown in the image output 153. Additionally, when the user moves either bar/cursor 600/602, a corresponding transverse cross-sectional images of vessel segment will be displayed in the image output 152 on the screen.

Another aspect of the method for vascular imaging co-registration may include estimating the accuracy of the imaging registration. As discussed herein, there may be some misalignment/error/discrepancy between the predicted and marked location(s) of detected anatomical landmark(s) on the extravascular imaging data, and the corresponding visualized anatomical landmark on the extravascular imaging data. As disclosed herein, one aspect of the method for vascular imaging co-registration may include aligning the predicted location of the detected anatomical landmark with the visualized anatomical landmark, and this may help alleviate some of this misalignment/error/discrepancy in the co-registration. However, the fact that this misalignment/error/discrepancy occurred in the first place (e.g. prior to aligning the predicted location of the detected anatomical landmark with the visualized anatomical landmark) may suggest a desire, and in some cases may provide a mechanism, to estimate the accuracy of the image co-registration. The system may include software or hardware that is configured to estimate the accuracy of the co-registration, and in some cases, display and/or otherwise indicate an estimated level of accuracy for portions of or all of the co-registration.

A variety of methods may be used to estimate the accuracy of the co-registration. For example, the error between predicted locations of detected anatomical landmark and the corresponding visualized anatomical landmarks can be measured, either individually, in groups, or as a whole over the entire co-registration. The magnitude these measurements may indicate an estimated level of accuracy of parts or all of the co-registration. For example, if the magnitude of measured error is large and/or exceeds certain predetermined thresholds, the predicted level of accuracy of the co-registration may be low. On the other hand, if the magnitude these measurements is large and/or exceeds certain predetermined thresholds, the predicted level of accuracy of the co-registration may be low. These estimations of accuracy may be made for portions of, or for the entre co-registration. Other example factors that may be used in estimating the accuracy of the co-registration may include: the total number actual/known locations or registration points used in the co-registration (e.g. obtained via fluoroscopy); the distance between actual/known locations or registration points; the total number of alignments performed (e.g. aligning a predicted location of the detected anatomical landmark with a visualized anatomical landmark); the distance between the performed alignments; and the degree of tortuosity of the vessel being analyze. Other methods of estimating accuracy may involve using curve-based algorithms or formulas, foreshortening predictions formulas or models, and the like.

The estimated level of accuracy of the co-registration may be performed for all, or portions of co-registration and/or over all or segments of the portion of the vessel being analyzed. Once an estimated level of accuracy is determined, the estimated level of accuracy for all, or portions of co-registration be displayed. For example, the system may include software or hardware that is configured to generate a visual indicator representing the estimated accuracy of the imaging co-registration. In some embodiments, the visual indicator may be displayed and/or overlaid on all or portions of the illustrated blood vessel on the extravascular imaging data. The visual characteristic may include color, symbol, intensity, or the like, and may be overlaid/superimposed on or associated with segments of the vessel shown. In some cases, different colors, symbols, intensity levels may be coded and/or used to indicate the level of accuracy. For example, if the level of accuracy is determined to be high for a particular segment, one color (e.g. green) may be overlaid on that segment. If alternatively, the level of accuracy is determined to be low for another particular segment, another color (e.g. red) may be overlaid on that segment. As can be appreciated, these are given by way of example only, and a broad variety of other configurations are contemplated.

In another aspect, it can be appreciated there may be error between an actual/known location and a calculated location, and this error may be determined or measured. In some embodiments, this error may be taken into account and used to adjust the co-registration. For example, an error/total travel distance ratio may be used as a scaling factor to recalculate and adjust previously calculated/predicted positions on the extravascular image (e.g. angiograph) for the entire preceding period in which the fluoroscope was inactive.

In some instances, it may be beneficial to use the co-registered intravascular imaging data and extravascular imaging data in determining a position of interest for performing a subsequent medical procedure or analysis. A position of interest may denote a particular location within a particular blood vessel, for example. A position of interest may define a particular starting point and a particular ending point (e.g. a range) within the particular blood vessel for performing a particular medical procedure or analysis. In studying the co-registered intravascular imaging data and extravascular imaging data to determine a position of interest, the physician or other professional may, as will be discussed, drop one or more markers or bookmarks onto the co-registered intravascular imaging data and extravascular imaging data in order to subsequently identify the position of interest.

Examples of subsequent medical procedures or analysis include, but are not limited to, angioplasty, atherectomy and/or stent implantation, physiological measurements such as pressure measurements including FFR (fractional flow reserve) measurements, iFR (instantaneous wave-free ratio) measurements, DFR (diastolic hyperemia-free ratio) measurements, and the like. The co-registered intravascular imaging data and extravascular imaging data may be used in guiding other diagnostic procedures such as but not limited to diagnostic angiography and coronary physiological examination. The co-registered intravascular imaging data and extravascular imaging data may be useful in reducing the usage of x-ray contrast, as the physician or other professional does not need to inject contrast to confirm a location of interest. In some instances, for example, a physician or other professional may study the intravascular imaging data, the extravascular imaging data, and even the co-registration between the intravascular imaging data and the extravascular imaging data (e.g. co-registered image data) to determine an appropriate location within a blood vessel of a patient for performing a subsequent medical procedure or analysis.

This may, for example, include having a patient visit a catheter lab, such as shown in the system 102 of FIG. 1, in order to obtain and co-register extravascular image data (e.g. angiogram/fluoroscopy) and intravascular image data (e.g. IVUS or OCT images) as described herein. The physician or other professional may subsequently study the data to determine if the patient is a candidate for a particular medical procedure or analysis, as well as to determine a position of interest, i.e., an optimal location, within the blood vessel for performing the particular medical procedure or analysis. If the physician or other professional determines that the patient is a candidate for implantation of a stent, the physician or other professional may use the co-registered data to determine where to implant the stent, thereby determining the position of interest. If the physician or other professional determines that the patient is a candidate for balloon angioplasty, for example, the physician or other professional may use the co-registered data to determine exactly where balloon angioplasty should be performed, thereby determining the position of interest. If the physician or other professional determines that the patient is a candidate for rotational atherectomy, the physician or other professional may use the co-registered data to locate the plaque that needs to be removed, thereby determining the position of interest. If the physician or other professional determines that the patient is a candidate for performing a particular medical procedure or analysis on the patient, the physician or other professional may use the co-registered data to determine one or more locations within the blood vessel for performing the particular medical procedure or analysis, thereby determining the position or positions of interest.

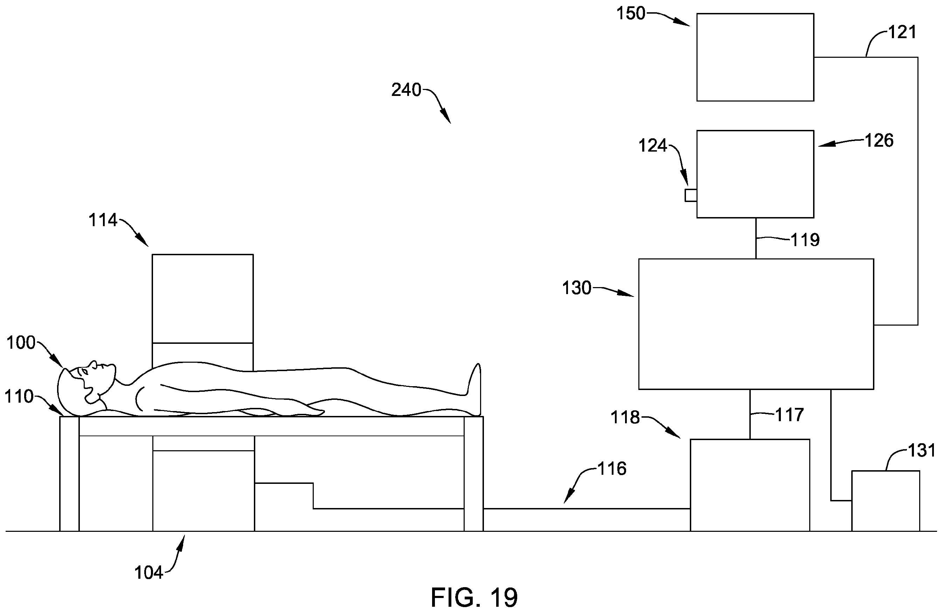

Once the physician or other professional has studied the co-registered data and determined a course of action for a particular patient, the physician or other profession may have the patient return to the catheter lab so that the subsequent procedure may be performed. FIG. 19 is a view of a catheter lab 240. The catheter lab 240 may be similar to the system 102 shown in FIG. 1, but in this case the intravascular sub-system 106, including an intravascular imaging device such as the imaging catheter 120, has been put away. The catheter lab 240 may include additional equipment (not shown) for performing any of a variety of different catheter-based interventions, such as stent implantation, balloon angioplasty, balloon cutting procedure, atherectomy, and others.

In some instances, once the physician or other professional has determined a course of action for their patient, including determining what medical procedure to perform and a general and/or exact location within the blood vessel where that procedure should be performed, thereby determining the position of interest, the physician or other professional may drop one or more bookmarks or markers onto the co-registered data in order to mark the position of interest. While the co-registered data may reside within the IVUS control unit 126, the computer 130 or the data archive or memory 131, the co-registered data, or at least portions thereof, may be communicated to the display 150 to be displayed for viewing by the physician or other professional. In some cases, the co-registered data may be displayed on a display that is part of an extravascular imaging system, such as a fluoroscopy system, or the like, for example.

In some instances, extravascular imaging data of a portion of a blood vessel may be obtained, the extravascular imaging data including an extravascular image showing an intravascular imaging device disposed within the vessel, with an imaging element of the intravascular imaging device disposed at a starting location for a translation procedure during which the imaging element is translated within the blood vessel from the starting location to an ending location. An extravascular contrast image showing the portion of the blood vessel with contrast and showing a visualized anatomical landmark is obtained. Intravascular imaging data is obtained from the intravascular imaging device during the translation procedure, the intravascular imaging data including one or more intravascular images showing a detected anatomical landmark. The detected anatomical landmark and the visualized anatomical landmark are used to co-register the intravascular imaging data with the extravascular imaging data as discussed herein. Subsequently, a position of interest within the vessel between the starting location and the ending location is determined and may be displayed on a live fluoroscopy session display. Determining the position of interest may include dropping one or more markers or bookmarks onto the co-registered intravascular imaging data and extravascular imaging data. Displaying the position of interest on the live fluoroscopy display may, for example, include displaying the one or more markers or bookmarks.

In some cases, a method for determining a position for performing a medical procedure includes obtaining extravascular imaging data of a portion of a blood vessel, the extravascular imaging data including an extravascular image showing a starting location and an ending location for a translation procedure during which an intravascular imaging element is translated within the blood vessel from the starting location to the ending location. Intravascular imaging data is obtained from the intravascular imaging device during the translation procedure. The intravascular imaging data is co-registered with the extravascular imaging data to create co-registered imaging data. The data may be co-registered, for example, using any of the co-registration methods described herein. In some cases, the co-registered intravascular imaging data and extravascular imaging data may be displayed. The co-registered imaging data is used to determine a position of interest within the portion of the blood vessel between the starting location and the ending location. The position of interest may be displayed, for example, on a live fluoroscopy session display.

In some instances, determining a position of interest within the vessel between the starting location and the ending location may include automatically determining a position of interest based on the intravascular imaging data. In some cases, this may include a process known as ALA (Automatic Lesion Assessment). Details and features regarding ALA are described for example in U.S. Patent Application No. 17/683,654, filed March 1, 2022; U.S. Patent Application No. 63/233,875, filed August 17, 2021; and 63/253,881, filed October 8, 2021. Each of these three applications are incorporated by reference herein. For example, one or more of the components of the system, such as the computer 130, may include software or hardware that is configured to perform image processing and/or image-recognition configured to identify one or more positions and/or locations of interest. Additionally, the software or hardware may be configured to mark and/or label the positions and/or locations of interest accordingly.

In other cases, the determination of the position of interest and/or the marking/labeling may be done manually by a user, for example, through a user interface. For example, the system software may allow for a user to place marks and/or labels onto the image in the desired locations through the use of a user interface. The physician or other professional may study the displayed co-registered intravascular imaging data and extravascular imaging data and manually identifying one or more positions of interest. In some instances, the physician or other professional may manually mark the position or positions of interest. This may include the physician or other professional manually dropping one or more markers or bookmarks onto the displayed co-registered intravascular imaging data and extravascular imaging data.

In some instances, and with reference to FIG. 19, displaying the position of interest on a live fluoroscopy display may further include providing the position of interest to a live fluoroscopy machine via a data link with the live fluoroscopy display. The data link may be represented by the transmission cable 121, for example. In some cases, the data link may be bi-directional, allowing communications in both directions. In some instances, a data security protocol may be implemented in order to ensure data integrity and to ensure patient privacy. In some cases, at least some of the elements of the catheter lab 240, such as but not including the computer 130 and the data storage 131, may be remote from the catheter lab 240. Accordingly, the data link may include a LAN (local area network) or a WAN (wide area network). In some cases, the data link may include the Internet.

Another example includes a method for determining a position of interest for performing a medical procedure or analysis. Examples of possible medical procedures include, but are not limited to, angioplasty, atherectomy, balloon cutting procedures and/or stent implantation. Examples of analyses that may be performed include physiological measurements such as pressure measurements including FFR (fractional flow reserve) measurements, iFR (instantaneous wave-free ratio) measurements, DFR (diastolic hyperemia-free ratio) measurements, and the like.

The method includes obtaining extravascular imaging data for a portion of a blood vessel and obtaining intravascular imaging data for the same portion of the blood vessel. The intravascular imaging data is co-registered with the extravascular imaging data. A position of interest for performing a medical procedure or analysis is determined and the position of interest is displayed on a live fluoroscopy session display. In some instances, determining the position of interest may include studying the co-registered intravascular imaging data and extravascular imaging data to determine one or more points of interest, and dropping one or more markers or bookmarks onto the co-registered intravascular imaging data and extravascular imaging data in order to indicate the position of interest. Displaying the position of interest on the live fluoroscopy display may, for example, include displaying the one or more markers or bookmarks.

Another example includes a method for determining a position of interest for implanting a stent. The method includes obtaining extravascular imaging data for a portion of a blood vessel and obtaining intravascular imaging data for the same portion of the blood vessel. The intravascular imaging data is co-registered with the extravascular imaging data. A desired implantation location for implanting a stent is determined, thereby identifying a position of interest, which is displayed on a live fluoroscopy session display. In some instances, determining the position of interest for implanting the stent may include studying the co-registered intravascular imaging data and extravascular imaging data to determine one or more points of interest, and dropping one or more markers or bookmarks onto the co-registered intravascular imaging data and extravascular imaging data in order to indicate the position of interest. In some instances, the points of interest may be determined automatically. In some cases, the points of interest may be determined manually. Displaying the position of interest on the live fluoroscopy display may, for example, include displaying the one or more markers or bookmarks.

Alternatively or additionally, displaying a desired position of interest may include displaying one or more of a distal marker that indicates a distal end of a position of interest and a proximal marker that indicates a proximal end of a position of interest. The position of interest may be considered as extending between the distal marker and the proximal marker, for example. A particular blood vessel may have one, two or more positions of interest, each located along the blood vessel. In some cases, each of the positions of interest within the blood vessel may include a distal marker and a proximal marker. In some cases, the markers indicating each of the positions of interest may be uniquely colored, in order to more easily see where a first position of interest is relative to a second position of interest, for example. In some cases, each distal marker may share a first color while each proximal marker may share a second color. For example, each distal marker may be green while each proximal marker may be yellow. A person viewing the markers will be able to identify the distal and proximal ends of each of the positions of interest.

In some cases, the position of interest may be displayed on live fluoroscopy in an intravascular imaging console via a real-time data connection from the fluoroscopy machine. The display of the position of interest may be automatically synchronized with cardiac motion by using ECC gating. In some cases, the display of the position of interest may be automatically synchronized with motion analysis of a radiopaque object such as a guide catheter or guide wire.

A position of interest, or two or more positions of interest, may each define a desired location for performing a medical procedure such as balloon angiography, rotational atherectomy. A position of interest may define a desired stent implantation location, for example. In some cases, a first position of interest within a particular blood vessel may be a candidate for a first medical procedure while a second position of interest within the same blood vessel, but spaced apart from the first position of interest, may be a candidate for a second medical procedure. As an example, a first position of interest may be a candidate for rotational atherectomy while a second position of interest may be a candidate for balloon angioplasty. In some cases, a position of interest may be a candidate for multiple medical procedures. As an example, a particular position of interest may be a candidate for either rotational atherectomy or balloon angioplasty, followed by stent implantation in order to maintain the patency of the blood vessel achieved during an initial procedure to open up the blood vessel. This is just an example.

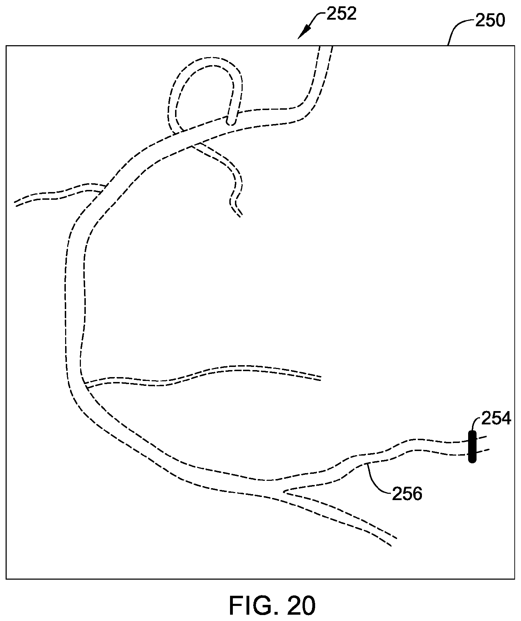

FIG. 20 is a schematic illustration of a display 250 showing an example live fluoroscopy image 252. It will be appreciated that the live fluoroscopy image 252 is similar to the angiographic/fluoroscopic image 151, showing the same portion of the patient's blood vessel as was previously captured in co-registered images. The live fluoroscopy image 252 includes a marker 254 that has been superimposed on a portion of a side branch 256. The marker 254 may have been placed on the co-registered data by the physician or other professional, or may have been automatically placed by the system via ALA, for example, and has been communicated to and superimposed on the live fluoroscopy image 252. In some instances, only a single marker 254 is needed. In cases where only a single marker 254 is used, the marker 254 may for example indicate a distal end of a position of interest, such as a treatment site. The marker 254 may indicate where a distal end of a stent should be located, or other treatment may be performed, for example. The marker 254 may indicate a distal-most end of a plaque to be removed by atherectomy, or perhaps where the distal end of a balloon for performing balloon angioplasty should be located prior to inflating the balloon. These are just examples, as other examples of potential vascular treatments are known and are contemplated.

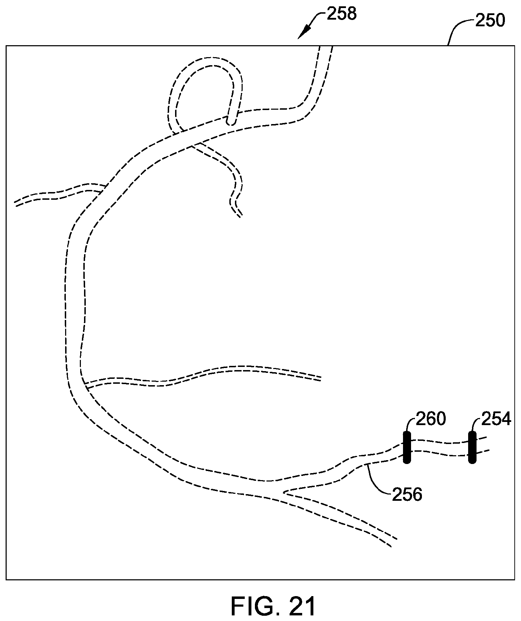

FIG. 21 is a schematic illustration of the display 250, showing an example extravascular image, such as a live fluoroscopy image 258. The live fluoroscopy image 258 is similar to the live fluoroscopy image 252, but includes the marker 254 and a second marker 260 that have been superimposed on the side branch 256. The marker 260 may have been placed on the co-registered data by the physician or other professional (e.g. through the system user interface), or automatically via ALA, for example, and has been communicated to and superimposed on the live fluoroscopy image 258. It will be appreciated that the marker 254 and the marker 260 in combination may mark a position of interest, which in this case is a treatment zone between the marker 254 and the marker 260. In some instances, the marker 254 may be considered as being a distal marker, for example, while the marker 260 may be considered as being a proximal marker.

If the patient is destined for a stent implantation, the marker 254 may indicate where the distal end of the stent should be located and the marker 260 may correspondingly indicate where the proximal end of the stent may be. The distance between the marker 254 and the marker 260 may indicate a region for performing any of a variety of other potential treatments, such as atherectomy, balloon angioplasty, cutting balloon treatment, physiological measurements, thrombectomy, additional imaging, or the like, for example.

When viewing the co-registered intravascular imaging data and extravascular imaging data, the physician or other professional may drop additional bookmarks or other markers in order to indicate one or more additional positions and/or spots of interest. These may include areas that the physician or other professional are not sure will require treatment, but wish to take a second look at under live fluoroscopy or other imaging or testing techniques. The physician or other professional may drop a first marker or bookmark to indicate that a first location within the blood vessel may require a first type, style or size of stent, for example, while a second location within the blood vessel may require a second type, style or size of stent. These markers or bookmarks may be in addition to the markers or bookmarks, either manually or automatically placed, that indicate distal and proximal landing zones. Examples of reasons for placing additional markers or bookmarks, which may be considered as being secondary markers or bookmarks (with those indicating the distal and proximal landing zones being considered as primary markers or bookmarks, include calcium locations and stent edges.

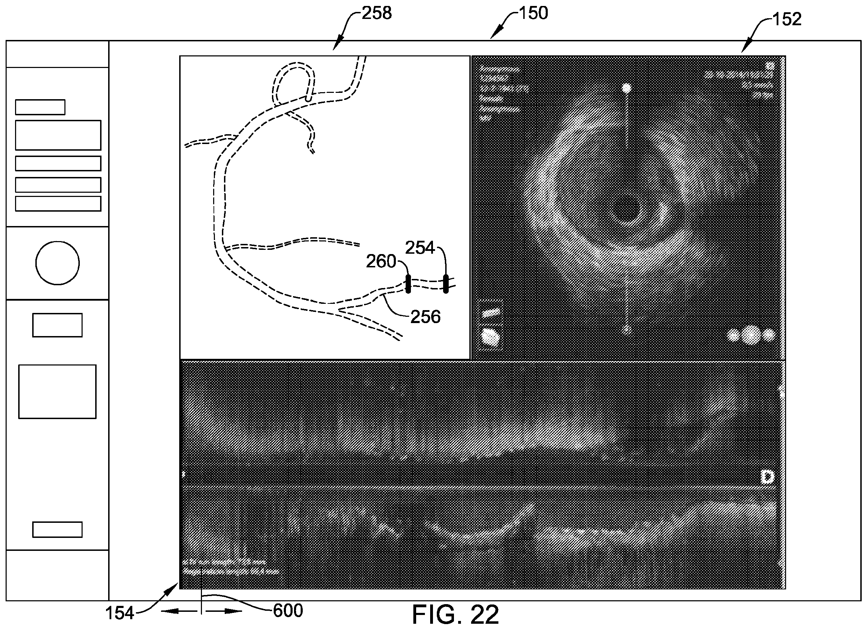

In some cases, it may be desirable to display the live fluoroscopy data in place of, or in addition to, the co-registered data that is shown for example in FIG. 18. FIG. 22 is similar to FIG. 18, but the co-registered data shown in FIG. 18 has been replaced with the live fluoroscopy image 258. As shown, this allows the physician or other professional to see the intravascular imaging data (IVUS data) on a screen at the same time as the live fluoroscopy image 258. In some cases, depending on how the screen is laid out, the live fluoroscopy image 258 may be displayed next to or otherwise in combination with the previous angiographic/fluoroscopic image 151.

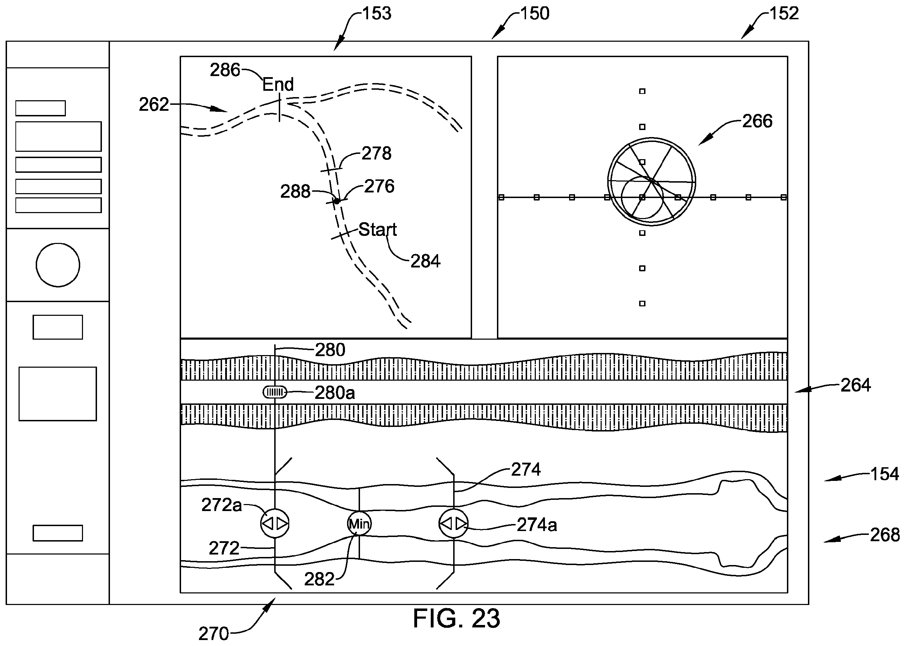

FIG. 23 shows an example of such a resulting co-registered display including both the extravascular images and the corresponding intravascular images. As can be appreciated, the co-registered extravascular and intravascular images can be simultaneously displayed, along-side one another, upon the display 150. For example, the display 150 includes image output 153 in the upper left portion for showing co-registered extravascular imaging data 262, image output 154 along the bottom for schematically showing co-registered longitudinal cross-sectional intravascular images 264 obtained during the translation procedure (e.g. from start to end), and image output 152 in the upper right portion for schematically showing corresponding co-registered transverse cross-sectional intravascular images 266 obtained during the translation procedure.

In some instances, as shown, the image output 154 includes a stylized representation 268 of the portion of the blood vessel schematically shown in the longitudinal cross-sectional intravascular images 264. As can be seen, the effective cross-sectional diameter of the blood vessel as shown in the stylized representation 268 varies as the anatomy of the blood vessel, including regions of plaque within the blood vessel, varies. In some instances, the stylized representation 268 may include numerical markings indicating an effective diameter of the blood vessel at a particular location, for example. In some embodiments, the system may include software or hardware that is configured to allow a user to scroll and/or track through the series of co-registered images. For example, the system may include a configuration that allows a user to scroll through one of the sets of co-registered images, and as the scrolling occurs, the processor acquires and displays corresponding co-registered images for the other sets of images.

For example, with reference to FIG. 23, an interactive slider 270 may be superimposed over the stylized representation 268 of the blood vessel. The interactive slider 270 includes a first slide bar 272 that may be used to indicate a starting point for a position of interest and a second slide bar 274 that may be used to indicate an ending point for a position of interest. In some cases, the first slide bar 272 may be used to indicate a distal point 276, as seen for example on the co-registered extravascular imaging data 262 shown in the image output 153. The second slide bar 274 may be used to indicate a proximal point 278, as seen for example on the co-registered extravascular imaging data 262 shown in the image output 153. Once the physician or other professional has placed the first slide bar 272 and the second slide bar 274 where the interactive slider 270 is marking a position of interest, the system may allow the physician or other professional to indicate to the system that they desire to place markers or bookmarks at these positions. The first slide bar 272 may include a first cursor button 272a that allows the physician or other professional to move the first slide bar 272, for example, by touching and dragging the first cursor button 272a. In some cases, the physician or other professional may move the first slide bar 272 using a mouse or keyboard, for example. The second slide bar 274 may include a second cursor button 274a that allows the physician or other professional to move the second slide bar 274, for example, by touching and dragging the second cursor button 274a. In some cases, the physician or other professional may move the second slide bar 274 using a mouse or keyboard, for example.

The interactive slider 270 includes a bar 280 that correlates a position of the first slide bar 272 relative to the schematic representation 268 with the co-registered longitudinal cross-sectional intravascular images 264. In some instances, the bar 280 includes a touch point 280a that may be used, similarly to the first cursor 272a, to move the position of the bar 280. Moving the bar 280 causes the first slide bar 272 to move in a similar fashion. As the interactive slider 270 is manipulated, such as by moving the first slide bar 272, it will be appreciated that the co-registered transverse cross-sectional intravascular images 266 shown in the image output 152 will change to correspond to the indicated position within the registered longitudinal cross-sectional intravascular images 264 shown in the image output 154. In some cases, a corresponding slider indicator 288 may appear on the co-registered extravascular imaging data 262 shown in the image output 153. In some cases, the interactive slider 270 may include a MIN indicator 282 that graphically illustrates a point within the displayed portion of the blood vessel having a minimal effective diameter. This may be a result of the native anatomy of the blood vessel, or may indicate a location in which accumulated plaque has narrowed the blood vessel.

Accordingly, the illustrated position of the interactive slider 270 may indicate a position of interest that is defined between the first slide bar 272 and the second slide bar 274. Similarly, the two corresponding markers 276 and 278 as shown on the co-registered extravascular imaging data 262 may be considered as defining a position of interest in which the physician or other professional believes that a medical procedure or analysis is warranted. In some cases, as shown, the co-registered extravascular imaging data 262 may include a start point 284 and an end point 286. The start point 284 and the end point 286 may represent the starting point and the ending point, respectively, for an IVUS pullback that generated the displayed data. It will be appreciated that the start point 284 and the end point 286 may indicate a range within which the physician or other professional is able to view the co-registered intravascular imaging data and extravascular imaging data.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

### The following aspects are preferred embodiments of the invention.

1. A method for determining a position of interest for performing a medical procedure, the method comprising:
   obtaining extravascular imaging data of a portion of a blood vessel, the extravascular imaging data including:
      an extravascular image showing a starting location and an ending location for a translation procedure during which an intravascular imaging element is translated within the blood vessel from the starting location to the ending location;
   obtaining intravascular imaging data from the intravascular imaging device during the translation procedure;
   co-registering the intravascular imaging data with the extravascular imaging data to create co-registered imaging data;
   using the co-registered imaging data to determine a position of interest within the portion of the blood vessel between the starting location and the ending location; and
   displaying the position of interest on a live fluoroscopy session display.
2. The method of aspect 1, wherein determining a position of interest within the vessel between the starting location and the ending location comprises automatically determining a position of interest based on the intravascular imaging data.
3. The method of any one of aspects 1 or 2, wherein displaying the position of interest on the live fluoroscopy session display comprises using a real-time data connection from a fluoroscopy machine.
4. The method of any one of aspects 1 to 3, wherein displaying the position of interest on the live fluoroscopy session display further comprises automatically synchronizing the position of interest with cardiac motion via ECC gating.
5. The method of any one of aspects 1 to 4, wherein displaying the position of interest on the live fluoroscopy session display further comprises automatically synchronizing the position of interest with motion analysis of a radiopaque object.
6. The method of any one of aspects 1 to 5, further comprising displaying the co-registered intravascular imaging data and extravascular imaging data.
7. The method of aspect 6, wherein determining a position of interest within the vessel between the starting location and the ending location comprises studying the displayed co-registered intravascular imaging data and extravascular imaging data.
8. The method of any one of aspects 1 to 7, wherein the medical procedure comprises implanting a stent.
9. The method of aspect 8, wherein displaying the position of interest on a live fluoroscopy display comprises displaying a distal marker indicating a target location for a distal end of the stent.
10. The method of any one of aspects 8 or 9, wherein displaying the position of interest on a live fluoroscopy display further comprises displaying a proximal marker indicating a target location for a proximal end of the stent.
11. The method of any one of aspects 1 to 10, wherein displaying the position of interest on a live fluoroscopy display further comprises providing the position of interest to the live fluoroscopy machine via a data link with the live fluoroscopy display.
12. The method of aspect 11, wherein the data link comprises a bi-directional data link.
13. The method of any one of aspects 1 to 12, further comprising communicating data from the live fluoroscopy display to a display that is displaying the co-registered intravascular imaging data and extravascular imaging data.
14. The method of any one of aspects 1 to 13, further comprising implementing a data security protocol in order to ensure data integrity and protect patient privacy.
15. The method of any one of aspects 11 to 14, wherein the data link comprises the Internet.

## Claims

1. A method for simultaneously displaying co-registered extravascular and intravascular images along-side one another on a display (150) for an intravascular imaging system (102), the method comprising:
obtaining intravascular image data from an intravascular imaging system/subsystem (106) and obtaining extravascular image data from an extravascular imaging system/sub-system (104), wherein the extravascular imaging data and the intravascular imaging data are obtained during a translation procedure;
co-registering the intravascular imaging data with the extravascular imaging data to create co-registered extravascular imaging data and co-registered intravascular imaging data;
displaying a first image output (153) showing the co-registered extravascular imaging data upon a display (150);
displaying a second image output (154) showing the co-registered intravascular imaging data upon the display (150); and
displaying a second slider bar or cursor (600) on the second image output (154) and a corresponding first slider bar or cursor (602) on the first image output (153).

2. The method of claim 1, wherein the second slider bar or cursor (600) is overlaid on the second image output (154).

3. The method of claim 1 or claim 2, wherein the second slider bar or cursor (600) and the first slider bar or cursor (602) are linked such that when a user selects and drags the first slider bar or cursor (602) on the co-registered extravascular imaging data of the first image output (153) the second slider bar or cursor (600) correspondingly slides along the corresponding co-registered intravascular imaging data of the second image output (154); and/or
wherein the second slider bar or cursor (600) and the first slider bar or cursor (602) are linked such that when a user selects and drags the second slider bar or cursor (600) on the co-registered intravascular imaging data of the second image output (154) the first slider bar or cursor (602) correspondingly slides along the corresponding co-registered extravascular imaging data of the first image output (153).

4. The method of any one of the preceding claims, wherein the first image output is displayed in an upper left portion of the display (150); and/or
wherein the second image output (154) is displayed along a bottom of the display (150).

5. The method of any one of the preceding claims, further comprising:
displaying a third image output (152) showing a co-registered transverse cross-sectional intravascular image obtained during the translation procedure, wherein when the user moves the first and/or second slider bar or cursor (600, 602), a corresponding transverse cross-sectional image is shown in the third image output (152),
preferably, wherein the third image output (152) is displayed in an upper right portion of the display (150).

6. The method of any one of the preceding claims, further comprising:
displaying a distal marker (254) and a proximal marker (260) in the first image output (153).

7. The method of any one of the preceding claims, further comprising:
automatically detecting an anatomical landmark in the co-registered intravascular imaging data;
marking a predicted location of the detected anatomical landmark on the extravascular imaging data; and
aligning the predicted location of the detected anatomical landmark with a corresponding visualized anatomical landmark.

8. The method of any one of the preceding claims, further comprising:
calculating a path that an imaging element (182) takes during the translation procedure; and
superimposing the path of the imaging element (182) upon the co-registered extravascular imaging data.

9. The method of any one of the preceding claims, further comprising:
determining a position of interest for performing a subsequent medical procedure or
analysis using the co-registered intravascular imaging data and/or the co-registered extravascular imaging data.

10. The method of any one of the preceding claims, wherein the co-registered extravascular imaging data is obtained by angiography or fluoroscopy; and/or wherein the co-registered intravascular imaging data is obtained by intravascular ultrasound - IVUS - or optical coherence tomograph - OCT.

11. A computer program comprising instructions which, when the program is executed by a computer, cause the computer to carry out the method of any one of claims 1 to 10.

12. A computer configured to execute the computer program of claim 11.
